# EUROPEAN PATENT SPECIFICATION

(11) **EP 1 764 411 B1**
(45) Date of publication and mention of the grant of the patent: **02.10.2013**
(21) Application number: 06015496.0
(22) Date of filing: 25.07.2006
(51) Int. Cl.: C12N 9/42, C12N 15/09, C11D 3/386

(54) **Alkaline xylanase**
Alkalische Xylanase
Xylanase active en milieu alcalin

(30) Priority: 26.07.2005 JP 2005216369
(43) Date of publication of application: 21.03.2007
(73) Proprietor: KAO CORPORATION, Tokyo 103-8210 (JP)
(72) Inventor: Hakamada, Yoshihiro, Tochigi 321-3497 (JP); Izawa, Yoshifumi, Tochigi 321-3497 (JP); Ogawa, Akinori, Tochigi 321-3497 (JP); Kawano, Takato, Tochigi 321-3497 (JP); Wada, Yasunao, Wakayama 640-8580 (JP); Shikata, Shitsuu, Wakayama 640-8580 (JP)
(74) Representative: Vossius & Partner

(56) References cited:
- WO-A-98/39403
- DATABASE Geneseq [Online] 22 April 2004 (2004-04-22), "Xylanase from an environmental sample seq id 164." XP002412914 retrieved from EBI accession no. GSN:ADJ34948 Database accession no. ADJ34948 -& DATABASE Geneseq [Online] 22 April 2004 (2004-04-22), "DNA encoding xylanase from an environmental sample seq id 163." XP002412948 retrieved from EBI accession no. GSN:ADJ34947 Database accession no. ADJ34947
- DATABASE GENESEQ 15 May 1996 (1996-05-15), "Xylanase gene" XP002412915 Database accession no. AAT16103
- DATABASE UniProt 7 March 2006 (2006-03-07), "Xylanase X" XP002412916 Database accession no. Q2I6W5
- DATABASE EMBL 1 February 2006 (2006-02-01), "Paenibacillus sp. BL11 xylanase X (xylX) gene, partial cds." XP002412917 Database accession no. DQ241676
- SUBRAMANIYAN S ET AL: "Cellulase-free xylanases from Bacillus and other microorganisms" FEMS MICROBIOLOGY LETTERS, AMSTERDAM, NL, vol. 183, no. 1, 1 February 2000 (2000-02-01), pages 1-7, XP003001364 ISSN: 0378-1097

## Description

### Technical Field

The present invention relates to a xylanase which is useful as an enzyme component of detergents, and to a gene encoding the xylanase.

### Background Art

Various detergents such as those for clothes contain hydrolases such as protease, amylase, and lipase in order to improve detergency. The term "cellulase" collectively refers to the enzymes that hydrolyze β-1,4 glycoside bonds in cellulose. Cellulase barely reacts with cellulose molecules which constitute the crystalline region-which serves as the skeleton of the fiber-but softens the gel structure of hydrated cellulose in the non-crystalline region, and releases any dirt confined in the gel structure. Thus, cellulase is very useful as a detergent enzyme for clothes. In recent years, Horikoshi *et al*. discovered alkaline cellulase originating from an alkaliphilic *Bacillus* microorganism (JP-B-50-28515, Horikoshi & Akiba, Alkalophilic Microorganisms, Springer, Berlin (1982)), and since then, different kinds of alkaline cellulase which can be incorporated into heavy-duty detergents for clothes have been developed (JP-B-60-23158, JP-B-6-030578, USP 4,945,053, and JP-A-10-313859).

One type of dirt or stain which is difficult to remove through laundry once deposited on clothes is that originating from vegetables or fruit. When vegetable- or fruit-originating stains are deposited on clothes, dye components thereof are bonded to the constituents of cell walls of the vegetable or fruit through covalent bond or physical bond. Since the dye-bonded cell-wall constituents of vegetables or fruit are deposited on cotton fiber of clothes, stains of such an origin are difficult to remove through washing. The cell wall is primarily constituted by hemicelluloses such as crystalline cellulose and xyloglycans (xylans). Therefore, with an aim to remove stains of these origins, detergents containing cellulase and several hemicellulases (such as xylanase) have been proposed (JP-A-11-500464).
If there is an enzyme which acts on the crystalline region of cellulose without giving damage to cotton fiber and which degrades xylan, such an enzyme could effectively remove vegetable- or fruit-originating stains and would be useful as a detergent component for clothes from the viewpoints of both cost and productivity. However, among different types of cellulases which are currently incorporated in detergents, none is known to possess xyloglycan-degrading activity (i.e., xylanase activity) or to act on the crystalline portion of cellulose.

Meanwhile, when clothes manufactured from fabrics of cellulose-based synthetic fiber (cotton rayon), flax, hemp, jute, rami, or a similar fiber undergo repeated washing, fiber filaments are mechanically broken or crushed, producing nappy, or fuzzy, fabric surfaces. In addition, in colored fabrics, color fading results. Thus, there still remains a need for detergents which, when employed in washing clothes, scarcely cause nappy fabric surfaces or color fading.

Meanwhile, in recent years, with the progress of gene technology, enzymes serving as a detergent component have now been mass-produced through gene recombination techniques. Alkaline cellulase, one of such enzymes, is not an exception, and many relevant genes have already been cloned, and nucleotide sequences thereof have been determined. Some of such genes and nucleotide sequences have actually been used in manufacture of detergents.

### Summary of the Invention

The present invention is directed to a protein described in any of the following (a), (b) and (c):
(a) a protein having an amino acid sequence of SEQ ID NO: 2;
(b) a protein which exhibits xylanase activity and has an amino acid sequence retaining 95% or higher identity with the amino acid sequence of SEQ ID NO: 2;
(c) a protein which exhibits xylanase activity and has an amino acid sequence retaining 90% or higher identity with the amino acid sequence of SEQ ID NO: 2,
wherein the protein of (b) and (c) is an alkaline xylanase exhibiting the following enzymological properties.
1. Action:
   Hydrolyzes β-1,4-glycoside bonds of xylan.
2. Substrate specificity:
   Degrades xylan and crystalline cellulose, in particular xylan.
3. Optimum pH for reaction:
   When subjected to a reaction at 40°C using xylan as the substrate, optimum pH for the reaction is approximately 8.5.
4. pH Stability:
   When subjected to a reaction for 20 hours at 5°C using xylan as the substrate, residual activity as measured at pH 4.0 to 10.5 is not less than 80% of the non-treatment activity.
5. Optimum reaction temperature:
   When subjected to a treatment for 20 minutes at pH 9.0 using xylan as the substrate, optimum reaction temperature is about 50°C.
6. Thermal stability:
   When subjected to a treatment for 20 minutes at pH 7.0 using xylan as the substrate, a residual activity of not less than 90% of the non-treatment activity is exhibited up to 50°C.
7. Molecular weight:
   Has a molecular weight of about 40 kDa as determined by SDS-polyacrylamide gel electrophoresis.
8. Has an optimum reaction pH of approximately 5.5 as determined in a reaction performed at 40°C using cellulose powder as a substrate.

The present invention is also directed to a gene encoding such a protein.

The present invention is also directed to a gene comprising a polynucleotide described in any of the following (a) to (c).
(a) A polynucleotide having a nucleotide sequence of SEQ ID NO: 1;
(b) A polynucleotide which hybridizes to a polynucleotide having a nucleotide sequence of SEQ ID NO: 1 under stringent conditions, and which encodes a protein that exhibits xylanase activity.
(c) A polynucleotide which has a nucleotide sequence retaining 80% or higher identity with the nucleotide sequence of SEQ ID NO: 1 and encodes a protein that exhibits xylanase activity, wherein the protein of (b) and (c) is an alkaline xylanase exhibiting the following enzymological properties:
   (1) action:
      hydrolyzes β-1,4-glycoside bonds of xylan;
   (2) substrate specificity:
      degrades xylan and crystalline cellulose;
   (3) optimum pH for reaction:
      when subjected to a reaction at 40°C using xylan as a substrate, optimum pH for the reaction is approximately 8.5;
   (4) pH stability:
      when subjected to a treatment for 20 hours at 5°C using xylan as a substrate, residual activity as measured at pH 4.0 to 10.5 is not less than 80% of the non-treatment activity;
   (5) optimum reaction temperature:
      when subjected to a reaction for 20 minutes at pH 9.0 using xylan as a substrate, optimum reaction temperature is about 50°C;
   (6) thermal stability:
      when subjected to a treatment for 20 minutes at pH 7.0 using xylan as a substrate, a residual activity of not less than 90% of the non-treatment activity is exhibited up to 50°C;
   (7) molecular weight:
      has a molecular weight of about 40 kDa as determined by SDS-polyacrylamide gel electrophoresis; and
   (8) an optimum reaction pH of approximately 5.5 as determined in a reaction performed at 40°C using cellulose powder as a substrate.

The present invention is also directed to a recombinant vector harboring the above gene, and to a transformant containing the recombinant vector.

The present invention is also directed to a method of producing xylanase, including culturing the transformant and collecting xylanase from the culture.

The present invention is also directed to a detergent composition which contains the above-mentioned alkaline xylanase or a protein exhibiting xylanase activity.

### Brief Description of the Drawings

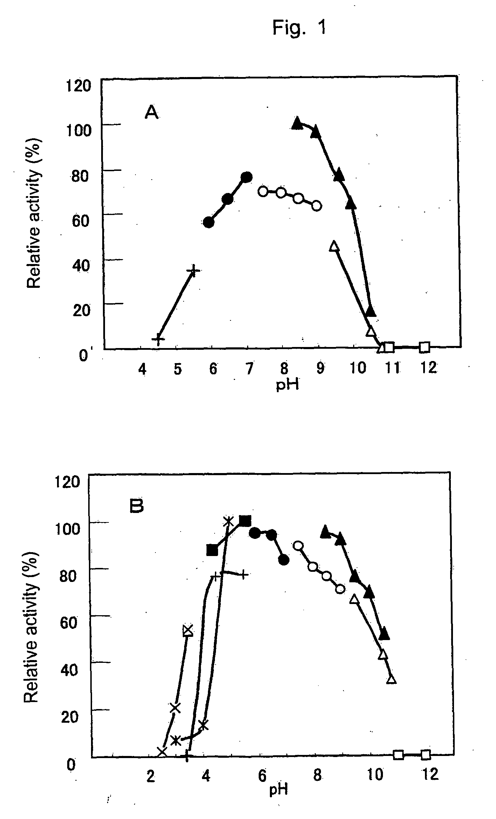

Fig. 1 includes graphs showing the optimum pH of the alkaline xylanase of the present invention, in which:
   A: xylanase activity, B: CP (cellulose powder) degrading activity, X: glycine-HCl buffer pH 2.5, pH 3, pH 3.5, *: citric acid - sodium citrate buffer pH 3, pH 4, pH 5, +: acetate buffer pH 3.5, pH 4.5, pH 5.5, solid black dots: phosphate buffer pH 6.0, pH 6.5, pH 7.0, white circles: Tris-HCl buffer pH 7.0, pH 8.0, pH 8.5, pH 9.0, solid black triangles: glycine sodium hydroxide pH 8.5, pH 9.0, pH 9.5, pH 10, pH 10.5, white triangles: sodium borate - sodium hydroxide buffer pH 9.5, pH 10.5, pH 10.8, white squares: phosphate sodium hydroxide buffer pH 11, pH 12.

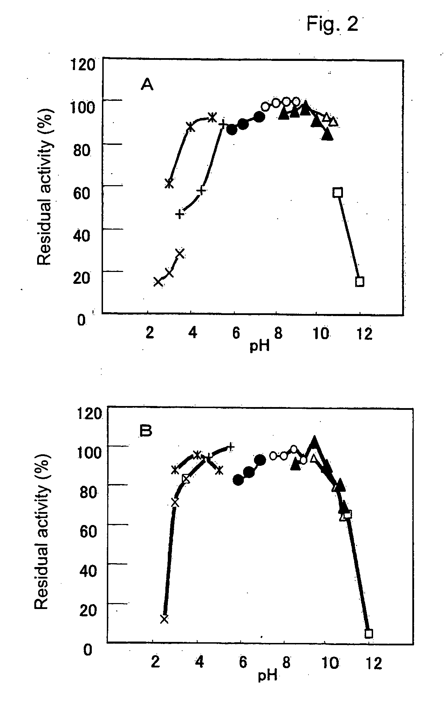

Fig. 2 includes graphs showing pH stability of the alkaline xylanase of the present invention, in which:
   A: xylanase activity, B: CP degrading activity, X: glycine-HCl buffer pH 2.5, pH 3, pH 3.5, *: citric acid - sodium citrate buffer pH 3, pH 4, pH 5, +: acetate buffer pH 3.5, pH 4.5, pH 5.5, solid black dots: phosphate buffer pH 6.0, pH 6.5, pH 7.0, white circles: Tris-HCl buffer pH 7.0, pH 8.0, pH 8.5, pH 9.0, solid black triangles: glycine sodium hydroxide pH 8.5, pH 9.0, pH 9.5, pH 10, pH 10.5, white triangles: sodium borate - sodium hydroxide buffer pH 9.5, pH 10.5, pH 10.8, white squares: phosphate sodium hydroxide buffer pH 11, pH 12.
Fig. 3 includes graphs showing the optimum temperature for reaction (hereinafter referred to as optimum reaction temperature) of the alkaline xylanase of the present invention, in which:
   A: xylanase activity, B: CP degrading activity.
Fig. 4 includes graphs showing the temperature stability of the alkaline xylanase of the present invention, in which:
   A: xylanase activity, B: CP degrading activity; solid black dots: 2mM calcium chloride was not added, white circles: 2mM calcium chloride was added.
Fig. 5 is a graph showing the effect of removing fuzziness from the surface of cotton cloths by the combination of the present alkalixylanase and cellulase (KSM-N145) .
   546b: N546b xylanase, N145: KSM-N145 cellulase
Fig. 6 is a graph showing the effect of removing fuzziness from the surface of cotton cloths by the combination of the present alkalixylanase and cellulase (Carezyme).
   Care: Carezyme (Novozyme), 546b: N546b xylanase

### Detailed Description of the Invention

The present invention provides an enzyme which acts within an alkaline pH region, exhibits xyloglycan degrading activity and crystalline-cellulose degrading activity, and thus is useful as a component of a detergent composition. The present invention also provides a gene encoding such an enzyme.
The present inventors conducted an extensive screening of naturally occurring alkaline-xylanase-producing microorganisms and identified a microorganism which produces an enzyme whose traits fit the above requirements, and by use of the thus-identified microorganism, the inventors successfully cloned a gene encoding alkaline xylanase, leading to completion of the present invention.

The enzyme of the present invention, called xylanase, is a novel enzyme, and is endowed with both xylanase activity and crystalline-cellulose hydrolyzing activity. The xylanase of the invention is useful as a component of a detergent for clothes and other items. In addition, through use of the alkaline xylanase gene of the present invention, the xylanase of the present invention alone can be produced on a large scale.

The alkaline xylanase of the present invention is a novel enzyme and has the following enzymological properties.

### (1) Action

Acts on xylan to thereby hydrolyze xylan.

### (2) Substrate specificity

Hydrolyzes xylan and crystalline cellulose (CP). Specifically, when individual substrates are caused to react with the alkaline xylanase in a 40°C glycine sodium hydroxide buffer (pH 9.0), the enzyme hydrolyzes xylan and CP. In particular, hydrolysis performance on xylan is remarkable. Meanwhile, carboxymethylcellulose (CMC), AZCL-Avicel, AZCL-curdlan, AZCL-mannann, and colloidal chitin are not hydrolyzed by the alkaline xylanase.

### (3) Optimum pH for reaction and pH dependency

When enzyme activity is measured under the below-described conditions and using xylan as a substrate, the optimum pH for reaction is in the vicinity of 8.5 or thereabouts. Between pH 8.0 and 9.0, not less than 90% of the maximum activity is exhibited, and between pH 7 and 10, not less than 60% of the maximum activity is exhibited (see Fig. 1A). Specifically, when xylan is the substrate in a reaction carried out at 40°C, the optimum pH for reaction (hereinafter referred to as optimum reaction pH) is approximately 8.5.

Meanwhile, when CP is the substrate, the optimum reaction pH is approximately 5.5, with not less than 90% of the maximum activity being exhibited between pH 4.5 and 9.0 and not less than 60% of the maximum activity being exhibited between pH 4 and 9.5 (see Fig. 1B). Thus, the alkaline xylanase of the present invention exhibits a very unique trait in that the enzyme has different optimum reaction pHs for different substrates.

### Measurement Conditions

Two different substrates, 1% (w/v) xylan and 1% (w/v) CP, are employed. Each substrate is added to each of the following 0.1M buffers, and reaction is allowed to proceed at 40°C for 20 minutes, whereby xylanase activity and CP degrading activity are determined.

Buffers: glycine-HCl buffer pH 2.5, pH 3, pH 3.5; citric acid - sodium citrate buffer pH 3, pH 4, pH 5; acetate buffer pH 3.5, pH 4.5, pH 5.5; phosphate buffer pH 6.0, pH 6.5, pH 7.0; Tris-HCl buffer pH 7.0, pH 8.0, pH 8.5, pH 9.0; glycine sodium hydroxide pH 8.5, pH 9.0, pH 9.5, pH 10, pH 10.5; sodium borate - sodium hydroxide buffer pH 9.5, pH 10.5, pH 10.8; and phosphate sodium hydroxide buffer pH 11, pH 12.

All activities are expressed as a percentage relative to the maximum activity (100%). In the measurement of xylanase activity, an enzyme solution (0.1 mL) of 0.1 U/mL in terms of xylan degrading activity was employed, whereas in the measurement of CP degrading activity, an enzyme solution (0.1 mL) of 0.25 U/mL in terms of CP degrading activity was employed.

### (4) pH Stability

When enzyme activity is measured under the below-described conditions, residual activities of xylanase activity and CP degrading activity are both 80% or more within a pH range of 4 to 10.5, proving that the xylanase of the present invention is stable over a wide pH range (see Fig. 2). Specifically, when xylan is used as a substrate and treatment is carried out at 5°C for 20 hours, residual activity, as determined within a pH range of 4.0 to 10.5, is not less than 80% of the activity of the untreated enzyme.

### Measurement Conditions

Using each of the following 0.1M buffers, the enzyme is treated at 5°C for 20 hours. Thereafter, enzyme activities are determined in accordance with the standard method for determining xylanase activity and the standard method for determining CPase activity, both described hereinlater under the heading "Referential Examples."

Buffers: glycine-HCl buffer pH 2.5, pH 3, pH 3.5; citric acid - sodium citrate buffer pH 3, pH 4, pH 5; acetate buffer pH 3.5, pH 4.5, pH 5.5; phosphate buffer pH 6.0, pH 6.5, pH 7.0; Tris-HCl buffer pH 7.0, pH 8.0, pH 8.5, pH 9.0; glycine sodium hydroxide pH 8.5, pH 9.0, pH 9.5, pH 10, pH 10.5; sodium borate - sodium hydroxide buffer pH 9.5, pH 10.5, pH 10.8; and phosphate sodium hydroxide buffer pH 11, pH 12.

All activities are expressed as a residual activity (%) relative to the activity of the pH-buffer-untreated enzyme (100%). In the measurement of xylanase activity, an enzyme solution of 0.1 U/mL in terms of xylanase activity was employed, whereas in the measurement of CP degrading activity, an enzyme solution of 0.25 U/mL in terms of CP degrading activity was employed.

### (5) Optimum reaction temperature

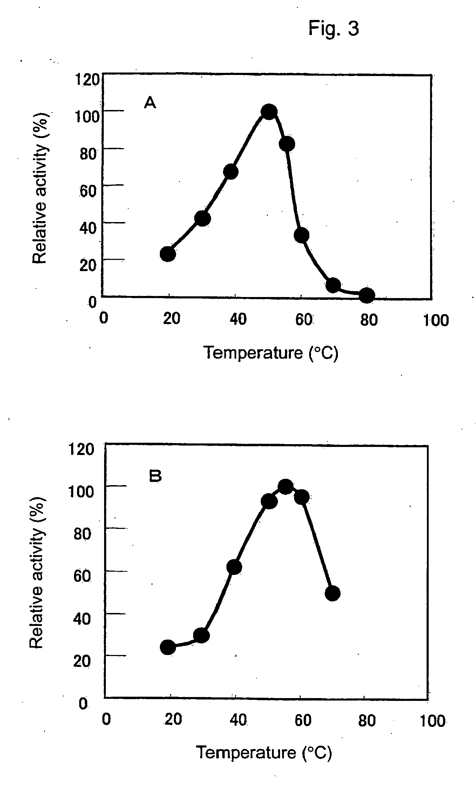

When enzyme activity is measured under the below-described conditions, the optimum reaction temperature that provides maximum xylanase activity is approximately 50°C, and not less than 60% of the maximum activity is exhibited between 40°C and 55°C (see Fig. 3A). Specifically, when xylan is used as a substrate and reaction is carried out at pH 9.0 for 20 minutes, the optimum reaction temperature is approximately 50°C.

The optimum reaction temperature that provides maximum CP degrading activity is approximately 55°C, and not less than 60% of the maximum activity is exhibited between 40°C and 60°C (see Fig. 3B).

### Measurement Conditions

In accordance with the standard methods for determining xylanase activity and CPase activity described in the Referential Examples hereinbelow, reaction is performed at different reaction temperatures; i.e., 20°C, 30°C, 40°C, 50°C, 55°C, 60°C, 70°C, and 80°C. All activities are expressed as a relative activity (%) to the value (100%) at a temperature at which a maximum activity is attained. In the measurement of xylanase activity, an enzyme solution (0.1 mL) of 0.05 U/mL in terms of xylan degrading activity was employed, whereas in the measurement of CP degrading activity, an enzyme solution (0.1 mL) of 0.125 U/mL in terms of CP degrading activity was employed.

### (6) Temperature stability

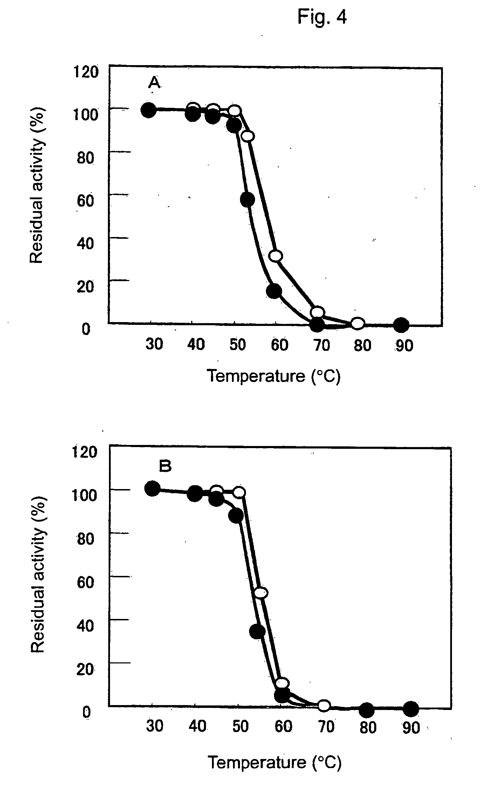

When enzyme activity is measured under the below-described conditions, residual activities of xylanase activity and CP degrading activity are both 90% or more after heat treatment at 50°C for 20 minutes, proving stability up to 50°C. In addition, 2mM calcium chloride slightly improves stability of the enzyme against heat (see Fig. 4). Specifically, when xylan is used as a substrate and reaction is carried out at pH 7.0 for 20 minutes, a residual activity of not less than 90% of the activity of the untreated enzyme is exhibited up to 50°C.

### Measurement Conditions

For the measurement of xylanase activity, an enzyme solution of 0.1 U (xylan degrading activity)/mL is employed, whereas for the measurement of CP degrading activity, an enzyme solution of 0.25 U (CP degrading activity)/mL is employed, and each of the enzyme solutions is treated with heat in a 0.1M phosphate buffer (pH 7.0) at several temperatures (30°C, 40°C, 50°C, 60°C, 70°C 80°C, 90°C) for 20 minutes. Thereafter, residual activities are determined in accordance with the standard method for determining xylanase activity and the standard method for determining CPase activity, both described hereinlater under the heading "Referential Examples." Residual activities are expressed as a percentage relative to the activity as measured without heat treatment (100%). The measurement was performed in parallel in two systems; i.e., a system where 2mM calcium chloride is added and a system where 2mM calcium chloride is not added.

### (7) Molecular weight

A molecular weight marker called "SDS-PAGE Standard LOW" (BioRad) was employed along with a gel (gel concentration: 12.5%), and electrophoresis was performed at 30mA for about 40 minutes. The electrophoresis gave a molecular weight of approximately 40 kDa. Since SDS-electrophoresis produces an error of about ±5000Da, the true value of molecular weight may vary within 40 kDa ±5000Da.

The protein (a) of the present invention having an amino acid sequence of SEQ ID NO: 2 is a novel xylanase which acts on xyloglycan to thereby degrade its β-1,4 glycoside bonds, and also on crystalline cellulose to thereby degrade its β-1,4 glycoside bonds. The protein according to the present invention encompasses not only the exact protein (a) having an amino acid sequence of SEQ ID NO: 2, but also other proteins equivalent to such a protein (may be referred to as "equivalent proteins") as described below.

When a protein of the present invention (i.e., alkaline xylanase; hereinafter may also be referred to as N546b xylanase) having an amino acid sequence of SEQ ID NO: 2 was compared with amino acid sequences of known enzymes in terms of identity, it was found that the highest identity (76%) was confirmed with a xylanase produced by *Bacillus* sp. strain YA355 (Ju-Hyun, Y. et al, J. Microbiol. Biotechnol., 3, 139-145, 1993). Also, a 76% identity was confirmed with a xylanase originating from *Bacillus* sp. 41M-1 (Nakai, et al., Nucleic Acids Symp. Ser., 31, 235-236, 1994), and a 51% identity was confirmed with a xylanase originating from a *Dictyoglomus thermophilum* strain. From the substrate specificity and the results of homology search, the enzyme of the present invention was considered to belong to the xylanase.
However, from the facts that the highest identity between the amino acid sequence of the alkaline xylanase of the present invention and amino acid sequences of other enzymes is 76%, and that, as described hereinbelow in the "Examples" section, the alkaline xylanase of the present invention has a conventionally unknown property of exhibiting different optimum pHs for different substrates, as proven by the optimum pH in relation to xylan degrading activity falling within the alkaline region and the optimum pH in relation to crystalline-cellulose-degrading activity using CP as the substrate falling within the acidic range, the alkaline xylanase of the present invention has been determined to be a novel enzyme.

Thus, a protein equivalent to the alkaline xylanase of the present invention is a protein (c) which exhibits xylanase activity and has an amino acid sequence of 90% or higher, even more preferably 95% or higher identity with the amino acid sequence of SEQ ID NO: 2, when the two sequences are appropriately aligned and which exhibits the above-described enzymological properties.

Homology of the above-mentioned amino acid sequences was determined by use of an amino acid sequence homology searcher employing BLAST 2 contained in the homology search tool provided by the Genome Net service developed by the Bioinformatics Center, Institute for Chemical Research, Kyoto University. The program employed was BLASTP, and the option parameters were initial settings (scoring matrix: BLOSUM62, no filter, alignment view: pairwise).

As used herein, exhibiting xylanase activity means exhibiting an activity of hydrolyzing β-1,4 glycoside bonds in xyloglycan. The level of this activity may not be particularly limited, so long as the mentioned hydrolysis performance is obtained. Thus, the level of activity may be similar to that of the protein having an amino acid sequence of SEQ ID NO: 2, or may be higher or lower than such a level.

The protein equivalent to the above-mentioned alkaline xylanase also exhibits a hydrolyzing activity on crystalline cellulose. By the expression "hydrolyzing activity on crystalline cellulose" is meant an activity of hydrolyzing β-1,4 glycoside bonds of crystalline cellulose. The level of this activity may not be particularly limited, so long as the mentioned hydrolysis performance is obtained. Thus, the level of activity may be similar to that of the protein having an amino acid sequence of SEQ ID NO: 2, or may be higher or lower than such a level.

Moreover, the protein equivalent to the above-mentioned alkaline xylanase may have additional traits. Such additional traits include possessing excellent stability as compared with a protein having the amino acid sequence of SEQ ID NO: 2, and exhibiting different functions or different optimum pHs but only when the temperature is low and/or high.

The alkaline xylanase of the present invention may be part of a larger protein, such as a fusion protein. Examples of a sequence which may be added to a fusion protein include a sequence useful in purification, such as a polyhistidinetag, and an additional sequence for securing stability during recombinant production.

When xylan is the substrate, the alkaline xylanase of the present invention exhibits an optimum pH for reaction of about 8.5, whereas when the substrate is cellulose powder (CP), the optimum pH is about 5.5. Thus, the xylanase of the present invention exhibits different optimum pHs for different substrates. Therefore, when the enzyme of the present invention is used as a component of a detergent, balance between xylanase activity and crystalline-cellulose degrading activity can be controlled by regulation of the pH of the environment in which the detergent is used.

The gene of the present invention encodes a protein having the amino acid sequence of SEQ ID NO: 2, and preferably, the gene is a polynucleotide (a) composed of a nucleotide sequence of SEQ ID NO: 1, or a gene equivalent thereto as described in the following. As used herein, the equivalent gene refers to a polynucleotide (b) which hybridizes to a polynucleotide having a nucleotide sequence of SEQ ID NO: 1 under stringent conditions, and which encodes a protein that exhibits xylanase activity; and a polynucleotide (c) which has a nucleotide sequence retaining 80% or higher identity with the nucleotide sequence of SEQ ID NO: 1 and encodes a protein that exhibits xylanase activity as described herein above.

As used herein, the term "stringent conditions" include' those conditions described, for example, in Molecular Cloning - A Laboratory Manual, 2nd edition (Sambrook, et al. 1989). Specifically, the stringent conditions may be such conditions in which a solution containing 6XSSC (composition of 1XSSC: 0.15M sodium chloride and 0.015M sodium citrate, pH 7.0), 0.5% SDS, 5X Denhardt solution, and 100-mg/mL herring sperm DNA is used together with probes, to thereby facilitate hybridization at a constant temperature of 65°C for 8 to 16 hours.

The gene equivalent to the gene (a) may have additional traits, such as a higher expression level of mRNA, higher stability, or more excellent stability of the protein obtained after translation, as compared with those of the gene (a).

In homology search of the above-mentioned nucleotide sequences, there was also employed the above-described homology searcher using BLAST 2 contained in the homology search tool provided by the Genome Net service developed by the Bioinformatics Center, Institute for Chemical Research, Kyoto University. The program employed was BLASTN, and the option parameters were initial settings (scoring matrix: BLOSUM62, no filter, alignment view: pairwise).

The alkaline xylanase gene of the present invention can be cloned from, for example, *Bacillus* sp. KSM-N546 (FERM P-19729), and cloning may be performed by use of any appropriate means, such as the shot-gun technique or PCR.

Also, the alkaline xylanase gene provided by the present invention can be created by altering a gene composed of a polynucleotide having a nucleotide sequence of SEQ ID NO: 1 through, for example, intentional or randomized mutagenesis.

For intentional mutagenesis, mutation may be designed in consideration of characteristic fragments in the nucleotide sequence. For example, such characteristic fragments may be determined on the basis of the projected spatial structure of of the protein of interest, or on the basis of the homology data with a known protein. Methods for achieving randomized mutagenesis include PCR and treatment with a mutagen. Methods for achieving intentional mutagenesis include site-specific mutagenesis. Specifically, there may be employed a kit named Site-Directed Mutagenesis System Mutan-SuperExpress Km Kit (Takara Bio Inc.). Alternatively, recombinant PCR (polymerase chain reaction) may be employed (PCR Protocols, Academic Press, New York, 1990).

Alternatively, the gene of the present invention can be obtained by acquiring, through any appropriate technique, a sequence fragment which hybridizes to a polynucleotide containing a portion of or the entirety of the gene of the present invention. Examples of the appropriate technique include PCR in which polynucleotides each containing a portion of the gene of the present invention are employed as a primer set, and a method in which polynucleotides each containing a portion of the gene of the present invention are employed as probes.

The alkaline xylanase of the present invention may be obtained by, for example, inoculating a microorganism capable of producing the enzyme in a natural or synthetic medium, culturing under routine culture conditions, and collecting the enzyme from the culture. Alternatively, the above-described enzyme gene may be artificially expressed and collected. Use of the present gene is advantageous in that the protein to be provided by the present invention can be mass-produced. An exemplary alkaline-xylanase-producing microorganism is *Bacillus* sp. KSM-N546 (FERM P-19729).

In order to produce alkaline xylanase through use of the gene of the present invention, the above-mentioned alkaline xylanase gene is inserted into any desired vector suitable for expressing the gene in a host of interest, and subsequently, through use of the resultant recombinant vector, a host is transformed, followed by culturing of the thus-obtained transformant. Alkaline xylanase can be collected from the culture. Culturing may be carried out in accordance with a routine method by inoculating the microorganism in a culture medium containing carbon source, nitrogen source, and other essential nutrients which are assimilable by the microorganism.

In order to create a recombinant vector harboring the alkaline xylanase gene of the present invention, the gene may be inserted into a vector which can securely maintain the gene, can maintain reproducibility within the host cell, and can successfully express the enzyme. When *Bacillus subtilis* is employed as the host, a DNA fragment derived from *Bacillus* sp. KSM-64 (FERM BP-2886), described in JP-A-2000-287687, may be linked to the alkaline xylanase gene to the upstream end thereof, so that alkaline xylanase can be expressed at a high level. Examples of the vector include pUC18, pBR322, and pHY300PLK when E. coli is the host, and pUB110, pHSP64 (Sumitomo, et al., Biosci. Biotechnol. Biocem., 59, 2172-2175, 1995) or pHY300PLK when *Bacillus subtilis* is the host.

The host is transformed with the thus-obtained recombinant vector through, for example, protoplast transformation, the competent cell method, or electroporation. No particular limitation is imposed on the host, and any organism may be employed. For example, there may be employed animals, cells originating from animals, plants, cells originating from plants, and microorganisms, wherein microorganisms are preferred. Preferred examples of microorganism hosts include Gram-positive bacteria such as those belonging to the genus *Bacillus* (*Bacillus subtilis*); Gram-negative bacteria such as *Escherichia coli*; and fungi belonging to the genus *Streptomyces* (actinomycetes), genus *Saccharomyces* (yiest), and genus *Aspergillus* (mold), with *Bacillus subtilis* being preferred, and *Bacillus subtilis* strains 168 and ISW1214 being particularly preferred. When cells of animal origin or plant origin are employed, preferably those belonging to cell lines that have been established as cultured cells are particularly preferred. Also, the host,is preferably a strain which lacks alkaline protease.

The thus-obtained transformant is cultured under appropriate conditions through use of a medium containing an assimilable carbon source, nitrogen source, metal salts, vitamins, etc. Conventional methods are applied to the resultant culture, whereby the enzyme can be collected and purified, and the purified enzyme may further be processed to have a desired form through freeze-drying, spray drying, and crystallization.

Collection and purification of the alkaline xylanase from the above-obtained culture may be performed in accordance with generally-employed methods. Specifically, cell bodies are removed from the culture through centrifugal separation or filtration, and the resultant culture supernatant is concentrated by use of routine means, whereby the enzyme of interest can be obtained. The-thus produced enzyme solution or dry powder, which may be used as is, may alternatively crystallized or granulated through known methods.

As described in the Examples section hereinbelow, the alkaline xylanase of the present invention is endowed with both xylanase activity and crystalline cellulose hydrolyzing activity. Therefore, the enzyme of the invention is useful as a component to be incorporated into a variety of detergent compositions.

In addition to alkaline xylanase of the present invention, the detergent composition of the present invention may further contain a variety of enzymes in combination. Examples of such enzymes include hydrolase, oxidase, reductase, transferase, lyase, isomerase, ligase, and synthetase. Among them, protease, cellulase, keratinase, esterase, cutinase, amylase, lipase, pullulanase, pectinase, mannanase, glucosidase, glucanase, cholesterol oxidase, peroxidase, and laccase are preferred, with protease, cellulase, amylase, and lipase being more preferred. More preferably, a cellulase capable of digesting crystalline cellulose is employed in combination of alkaline xylanase.

As described in the Examples section provided hereinbelow, combination of the present enzyme with a cellulase which degrades crystalline cellulose enables effective removal of "nappy" fibers from the fabric surfaces of cotton clothes. Accordingly, the use of this combination enables also to prevent color fade caused by the generation of nappy fiber in colored clothes. Examples of such a cellulase capable of degrading crystalline cellulose include KSM-S237 cellulase (JP-A-10-313859), KSM-N252 cellulase (JP-A-2001-340074), KSM-N145 cellulase (JP-A-2005-287441), Carezyme (Registered trade mark, product of Novozyme), etc.

The detergent composition of the present invention may contain any of known detergent components. Examples of such components include surfactants, divalent metal-ion trapping agents, alkali agents, anti-redeposition agents, bleaching agents, builders which are known in the laundry detergent field, softening agents, reducing agents (e.g., sulfurous acid salts), defoaming agents (e.g., silicone), perfumes, and other additives.

The detergent composition preferably contains a surfactant in an amount of 0.5 to 60 mass%. When the detergent composition is provided in the form of powder, the surfactant is preferably incorporated in an amount of 10 to 45 mass%, whereas when the detergent composition is provided in the form of liquid, the surfactant is preferably incorporated in an amount of 20 to 50 mass%.
Examples of the surfactant include anionic surfactants, nonionic surfactants, amphoteric surfactants, and cationic surfactants. These may be used singly or in combination. Preferably, an anionic surfactant or a nonionic surfactant is employed.

Preferred examples of the anionic surfactant include C10-C18 alcohol sulfuric acid ester salts, C8-C20 alkoxy alcohol sulfuric acid ester salts, alkylbenzenesulfonic acid salts, paraffinsulfonic acid salts, α-olefinsulfonic acid salts, α-sulfo fatty acid salts, α-sulfo fatty acid alkyl ester salts, and fatty acid salts. In the present invention, linear alkylbenzenesulfonic acid salts having a C10-C14 (more preferably C12-C14) alkyl group are particularly preferred. The counter ionic species is preferably an alkali metal ion or an amine ion, particularly preferably a sodium ion and/or a potassium ion; a monoethanolamine ion; or a diethanolamine ion.

Preferred examples of the nonionic surfactant include polyoxyalkylene C8-C20 alkyl ethers, alkyl polyglycosides, polyoxyalkylene C8-C20 alkylphenyl ethers, polyoxyalkylene sorbitan C8-C22 fatty acid esters, polyoxyalkylene glycol C8-C22 fatty acid esters, and polyoxyethylene-polyoxypropylene block polymers. The nonionic surfactant is particularly preferably a polyoxyalkylene alkyl ether obtained through addition of an alkylene oxide such as ethylene oxide or propylene oxide (4 to 20 mol) to a C10-C18 alcohol, the polyoxyalkylene alkyl ether having an HLB value (calculated by the Griffin method) of 10.5 to 15.0, preferably 11.0 to 14.5.

A divalent metal-ion trapping agent is incorporated in an amount of 0.01 to 50 mass%, preferably 5 to 40 mass%. Examples of the divalent metal-ion trapping agent include condensed phosphoric acid salts such as tripolyphosphoric acid salts, pyrophosphoric acid salts, and orthophosphoric acid salts; aluminosilicates such as zeolite; synthetic layered crystalline silicic acid salts; nitrilotriacetic acid salts; ethylenediaminetetraacetic acid salts; citric acid salts; isocitric acid salts; and polyacetal carboxylic acid salts. Of these, crystalline aluminosilicates (synthetic zeolite) are particularly preferred. Among A-type, X-type, and P-type zeolites, an A-type zeolite is preferred. The preferably employed synthetic zeolite has an average primary particle size of 0.1 to 10 µm, more preferably 0.1 to 5 µm.

An alkaline agent is incorporated in an amount of 0.01 to 80 mass%, preferably 1 to 40 mass%. When the detergent composition is provided in the form of powder, examples of the alkaline include alkali metal carbonates such as sodium carbonate, which is generally called dense ash or light ash, and amorphous alkali metal silicates of JIS No. 1, 2, or 3. These inorganic alkaline agents are effective in forming particle cores upon drying of a detergent to be able to provide a comparatively hard detergent having excellent fluidity. In place of these alkaline agents, for example, sodium sesquicarbonate or sodium hydrogencarbonate may be used, and a phosphoric acid salt such as a tripolyphosphoric acid salt also acts as an alkaline agent. When the detergent composition is provided in the form of liquid, examples of the alkaline agent to be employed which may act as a counter ion to a surfactant include sodium hydroxide and mono-, di-, or triethanolamine, as well as the aforementioned alkaline agents.

An anti-redeposition agent is incorporated in an amount of 0.001 to 10 mass%, preferably 1 to 5 mass%. Examples of the anti-redeposition agent include polyethylene glycol, a carboxylic acid polymer, polyvinyl alcohol, and polyvinylpyrrolidone. Of these, a carboxylic acid polymer has metal-ion trapping ability and ability to disperse solid particulate soils from clothes to a washing bath, as well as anti-redeposition ability. The carboxylic acid polymer is a homopolymer or copolymer formed of acrylic acid, methacrylic acid, itaconic acid, etc., and the copolymer is preferably formed through copolymerization of the aforementioned monomer with maleic acid. The molecular weight of the copolymer is preferably some thousands to 100,000. In addition to the aforementioned carboxylic acid polymer, a polymer such as a polyglycidic acid salt, a cellulose derivative such as carboxymethyl cellulose, or an aminocarboxylic acid polymer such as polyaspartic acid is preferably employed, since these substances also have metal-ion trapping ability, dispersibility, and anti-redeposition ability.

A bleaching agent (e.g., hydrogen peroxide or a percarbonate) is preferably incorporated in an amount of 1 to 10 mass%. In the case where a bleaching agent is employed, a bleach-activator such as tetraacetylethylenediamine (TAED) or one described in JP-A-1994-316700 may be incorporated in an amount of 0.01 to 10 mass%.

Examples of the fluorescent agent include biphenyl fluorescent agents (e.g., Cinopal CBS-X) and stilbene fluorescent agents (e.g., DM-type fluorescent dyes). Such a fluorescent agent is preferably incorporated in an amount of 0.001 to 2 mass%.

The detergent composition of the present invention can be produced through a customary method using the alkaline xylanase of the present invention in combination with the aforementioned known detergent components. The form of the detergent may be appropriately determined in accordance with use thereof, and the detergent may assume the form of, for example, liquid, powder, granule, paste, or solid.

The thus-produced detergent composition can be employed as, for example, a powder laundry detergent, a liquid laundry detergent, a detergent for an automatic dishwasher, and a detergent for modifying cotton fiber.

### Examples

### Example 1 Screening of alkaline-xylanase producing bacteria

Soil samples were collected from a number of sites in Japan, and each sample was suspended in sterilized water. The suspension was heated at 80°C for 30 minutes, and applied onto an agar plate medium having the following composition shown in Table 1. The medium was subjected to stationary culture in an incubator at 30°C for 3 days so as to grow bacteria. A medium in which peripheral transparent zones due to decomposition of xylan had been detected was selected, and production of single colonies was repeated, to thereby obtain *Bacillus* sp. KSM-N546 strain.

**[Table 1]**

| Composition of screening medium | |
|---|---|
| | (w/v) |
| Sodium chloride | 1.0% |
| Sodium carbonate (separately sterilized) | 0.5% |
| Potassium dihydrogenphosphate | 0.1% |
| Meat extract (Oxoide) | 1.0% |
| Bactopeptone (DIFCO) | 1.0% |
| Xylan (Oat-spelt, product of Fluka) | 1.0% |

### Example 2 Production and purification of alkaline xylanase derived from Bacillus sp. KSM-N546 strain (N546b xylanase)

The *Bacillus* sp. KSM-N546 strain was placed in a liquid medium having the composition shown in Table 2, followed by shake-culturing at 30°C for 3 days.

**[Table 2]**

| Medium for producing N546b xylanase | |
|---|---|
| | (w/v) |
| POLYPEPTON-S (NIHON PHARMACEUTICAL CO., LTD) | 2.0% |
| (Wako Fish meat extract Pure Chemical Industries, Ltd.) | 1.0% |
| Potassium monohydrogenphosphate | 0.15% |
| Yeast extract (DIFCO) | 0.1% |
| Magnesium sulfate heptahydrate | 0.07% |
| Carboxymethyl cellulose | 0.1% |
| Sodium carbonate (separately sterilized) | 0.5% |

The thus-obtained culture supernatant was diluted to about ten-fold with 50mM Tris-HCl buffer (pH 7.5). The diluted supernatant was applied onto SuperQ-Toyopearl 650M (Toso) equilibrated in advance with 50mM Tris-HCl buffer (pH 7.5), and eluted with 50mM Tris-HCl buffer (pH 7.5) (480 mL), whereby a non-adsorbed fraction containing alkaline xylanase was obtained. Subsequently, the non-adsorbed fraction was concentrated to 1/20 in volume by means of a PM10 concentration membrane (product of Amicon) while 10mM boric acid - sodium hydroxide buffer (pH 9.5) was added to the eluate. The concentrated product was applied onto a DEAE-Toyopearl 650M column (1 cm × 10 cm) equilibrated in advance with 10mM boric acid - sodium hydroxide buffer (pH 9.5), whereby alkaline xylanase was adsorbed onto the column. Adsorbed proteins were eluted sequentially with 10mM boric acid - sodium hydroxide buffer (pH 9.5) (200 mL) and 0 to 0.1M KCl (200 mL). Fractions exhibiting alkaline xylanase activity were collected, and the combined fraction was desalted and concentrated by means of a PM10 concentration membrane. Subsequently, the obtained concentrate was applied onto a CM-Toyopearl 650M column (1 cm x 10 cm) which had been equilibrated in advance with 10mM phosphate buffer (pH 6.0). Alkaline xylanase was eluted into a non-adsorbed fraction, and the fraction was desalted and concentrated by means of a PM10 concentration membrane. Finally, the concentrate was applied onto a hydroxyapatite column (1 cm x 10 cm) equilibrated in advance with 10mM phosphate buffer (pH 7.0). Alkaline xylanase was eluted into a non-adsorbed fraction, and the fraction was desalted and concentrated by means of a PM10 concentration membrane, whereby an enzyme sample purified to an electrophoretically homogeneous level was obtained. The purified sample was subjected to N-terminal amino acid sequence analysis.

### Example 3 Production of a gene fragment coding for N546b xylanase

### (1) Treatment of a genomic DNA with a restriction enzyme

A gene coding for N546b xylanase was obtained by means of an "LA PCR in vitro Cloning Kit" (product of Takara).

A genomic DNA was prepared from *Bacillus* sp. KSM-N546 obtained in Example 2 through the method described by Saito and Miura in Biochim. Biophys. Acta, 72, 619-629, 1963. Firstly, the genomic DNA of the KSM-N546 strain was treated with HindIII. Specifically, the N546 strain genomic DNA (2 µL, 1 µg), a 10 x buffer (10 µL), deionized water (24 µL), and HindIII (2 µL, 20 units) were mixed, and the mixture was treated at 37°C for 3 hours. After completion of reaction, the reaction mixture was purified by means of a "GFX PCR DNA and Gel Band Purification Kit" (product of Pharmacia), to thereby produce a purified DNA solution (25 µL).

### (2) Ligation reaction

To the purified DNA solution (20 µL), HindIII cassette (5 µL, 100 ng of DNA, a component of the LA PCR in vitro Clonig Kit) and Ligation High (25 µL) were added. The mixture was left to stand at 16°C overnight, and treated by means of a "GFX PCR DNA and Gel Band Purification Kit," to thereby purify and recover DNA.

### (3) Amplification of a gene fragment coding for N546b xylanase through PCR

A 10 x buffer (5 µL), LA Taq (0.5 µL), a dNTP mix (8 µL), MgCl₂ (5 µL), primer C1 (1 µL, 10 pmol) (these being components of the LA PCR in vitro Clonig Kit), and complex primer I (SEQ ID NO: 3) (4 µL), which had been deduced on the basis of the N-terminal sequence of the purified N546b xylanase, were added to the purified solution (26.5 µL) obtained in the above ligation step. The PCR conditions were as follows: heat denaturation at 94°C for 2 minutes, followed by 30 cycles of treatment each consisting of 94°C for 30 seconds, 55°C for 2 minutes, and 72°C for 2 minutes. The second PCR was performed by use of the above-obtained PCR solution (1 µL) serving as a template. Specifically, a 10 × buffer (5 µL), LA Taq (0.5 µL), a dNTP mix (8 µL), MgCl₂ (5 µL), cassette primer C2 (1 µL, 10 pmol), deionized water (26.5 µL) (these being components of the LA PCR in vitro Clonig Kit), and mixed primer II (SEQ ID NO: 4) (4 µL, 10 pmol), which had been deduced on the basis of the N-terminal sequence of the purified N546b xylanase, were added to the template DNA solution (1 µL). The PCR conditions were as follows: heat denaturation at 94°C for 1 minute, followed by 30 cycles of treatment each consisting of 94°C for 30 seconds, 55°C for 2 minutes, and 72°C for 1 minute. The resultant partially amplified fragment (about 1.2 kb) of a gene coding for N546b xylanase was analyzed in terms of partial nucleotide sequence by use of cassette primer C2 serving as a primer.

### Example 4 Determination of the total nucleotide sequence coding for an N546b xylanase gene through inverse PCR

Primers III to VI (SEQ ID NOs: 5 to 8) employed in inverse PCR were constructed on the basis of the nucleotide sequence which had been determined from partially amplified fragment (about 1.2 kb) of a gene coding for 546b xylanase. Subsequently, inverse PCR was performed by use of the primers. Firstly, a KSM-N546b strain genomic DNA solution (5 µL, 1 µg of DNA), a 10 × buffer (10 µL), deionized water (82 µL), and SacI (3 µL, 30 units) were mixed, and the genomic DNA was digested by the restriction enzyme at 37°C for 3 hours. The thus-digested genomic DNA was purified by means of a GFX PCR DNA and Gel Band Purification Kit, to thereby obtain a target product (20 µL). The product was subjected to self ligation with Ligation High (16°C, 18 hours). Subsequently, the first PCR was performed by use of the obtained ligation mixture (1 µL) serving as a template DNA and the aforementioned primers III (SEQ ID NO: 5) and IV (SEQ ID NO: 6). Specifically, a template DNA solution (1 µL), primers (5 µL each, 1 µM each), a 10 × buffer (5 µL), LA Taq (0.5 µL), a dNTP mix (8 µL), MgCl₂ (5 µL), and deionized water (21.5 µL) were mixed, and the mixture was subjected to PCR by means of a Gene Amp PCR System 9700 (product of Amersham Pharmacia). The reaction conditions were as follows: heat denaturation at 94°C for 2 minutes, followed by 28 cycles of treatment each consisting of 94°C for 1 minute, 55°C for 1 minute, and 72°C for 1 minute were repeated, and then 72°C for 1 minute. The PCR product was purified by means of a GFX PCR DNA and Gel Band Purification Kit, to thereby obtain an amplified DNA fragment (about 5.5 kbp).

Subsequently, the second inverse PCR was performed by use of the amplified DNA fragment serving as a template and the aforementioned primers V and VI. Specifically, a template DNA solution (0.5 µL), primers (5 µL each, 1 µM each), a 10 × buffer (5 µL), LA Taq (0.5 µL), a dNTP mix (8 µL), MgCl₂ (5 µL), and deionized water (19 µL) were mixed, and the mixture was subjected to PCR by means of Gene Amp PCR System 9700. The reaction conditions were as follows: heat denaturation at 94°C for 2 minutes, followed by 28 cycles of treatment each consisting of 94°C for 30 seconds, 57°C for 1 minute, and 72°C for 2.5 minutes, and then, 72°C for 2.5 minutes. The PCR product was purified by means of a GFX PCR DNA and Gel Band Purification Kit, followed by 1% (w/v) agarose gel electrophoresis, whereby an amplified DNA fragment (about 5.5 kb) was recovered from the gel. After purification, the DNA fragment was analyzed by use of primers V (SEQ ID NO: 7) and VI (SEQ ID NO: 8), whereby the total nucleotide sequence (about 1.8 kbp) of an N546b xylanase gene was determined.

### Examples 5 Subcloning of an N546b xylanase gene

Primers VII and VIII (SEQ ID NOs: 9 and 10) were constructed on the basis of a nucleotide sequence which is present upstream with respect to the promoter of the nucleotide-sequence-analyzed N546b xylanase gene, and a nucleotide sequence which is present downstream with respect to the terminator of the same gene, respectively. Subsequently, genome PCR was performed by use of the N546 strain genome serving as a template. Specifically, the N546 strain genome (0.5 µL, 1 µg), primer VII (having an added BglII recognition site, SEQ ID NO: 9) (5 µL), primer VIII (having an added HindIII recognition site, SEQ ID NO: 10) (5 µL), a 10 × buffer (5 µL), LA Taq (0.5 µL), dNTP mix (8 µL), MgCl₂ (5 µL), and deionized water (21 µL) were mixed, and the mixture was subjected to PCR by means of a Gene Amp PCR System 9700. The reaction conditions were as follows: heat denaturation at 94°C for 1 minute, followed by 28 cycles of treatment each consisting of 98°C for 10 seconds and 68°C for 4.5 minutes. The PCR product was purified with a GFX PCR DNA and Gel Band Purification Kit, to thereby obtain a target product (87 µL). Subsequently, a 10 × buffer (10 µL) and HindIII (3 µL, 30 units) were added to the product, and the system was treated with the enzyme at 37°C for 20 hours. The HindIII-treated system was purified by means of a GFX PCR DNA and Gel Band Purification Kit, followed by treatment with BglII in a similar manner. After further purification, the thus-obtained fragments contained in the system were ligated by use of Ligation High with shuttle vector pHY300PLK which had been treated with HindIII and BglII (16°C, 20 hours).

Subsequently, by use of the above-obtained ligation mixture (3 µL), competent cells E. coli JM109 (product of Takara) (100 µL) were transformed, whereby transformants having tetracycline resistance were obtained. Among the transformants, those harboring pHY300PLK (pHY546b) having a target N546b xylanase gene were selected through colony PCR. The selected transformants were treated by means of a Micro Prep Plasmid Purification Kit (product of Amersham Pharmacia) for recovery and purification of the plasmid of interest. Subsequently, *Bacillus subtilis* ISW1214 strain (a host bacterium) (product of Yakult) was transformed with the pHY546b through the protoplast method, whereby a transformant was obtained. The transformant was grown in a DM3 agar medium (Table 3) containing tetracycline hydrochloride (product of Sigma) (30 µg/mL).

**[Table 3]**

| DM3 agar medium | |
|---|---|
| Agar (Wako Pure Chemical Industries, Ltd.) | 0.8% (w/v) |
| Sodium succinate (Wako Pure Chemical Industries, Ltd.) | 0.5 M |
| Casamino acid (product of DIFCO) | 0.5% (w/v) |
| Yeast extract (product of DIFCO) | 0.5% (w/v) |
| Monopotassium phosphate | 0.35 (w/v)% |
| Dipotassium phosphate | 0.15 (w/v)% |
| Glucose | 0.5 (w/v)% |
| Bovine serum albumin (product of Sigma) | 0.01 (w/v)% |
| Magnesium chloride | 20 mM |
| CMC | 0.5% (w/v) |
| Trypan Blue | 0.02 (w/v)% |

### Example 6 Production of N546b xylanase through a recombinant technique

The transformant, which had been grown in a DM3 agar medium, was cultured (30°C, 72 hours, 125 rpm) in an LB medium (1% Bactotrypton, 0.5% yeast extract, 1% sodium chloride), followed by centrifugation (8000 rpm, 20 minutes, 4°C), to thereby obtain a culture supernatant (a crude N546b xylanase solution produced through a recombinant technique). The supernatant was found to exhibit xylanase activity of 41 units and a CP degrading activity of 0.43 units.

### Example 7 Properties of N546b xylanase

### (1) Substrate specificity

Substrate hydrolyzing ability of the thus-obtained N546b xylanase was determined in accordance with the methods described in the Referential Examples hereinbelow. The substrates tested were CMC, CP, xylan, AZCL-avicel, AZCL-curdlan, AZCL-mannan, and colloidal chitin. The enzyme was found to exhibit CP degrading activity of 0.36 with respect to the xylan-hydrolyzing activity (regarded as 100). No hydrolyzing activity was observed against CMC, AZCL-avicel, AZCL-curdlan, AZCL-mannan, or colloidal chitin.

### (2) Optimum reaction pH

Optimum reaction pH of the N546b xylanase was determined using 1% (w/v) xylan or 1% (w/v) CP as a substrate. The N546b xylanase was reacted with each substrate at 40°C for 20 minutes in various buffers (0.1 M each) which were glycine-HCl buffer (pH 2.5, pH 3, and pH 3.5), citric acid - sodium citrate buffer (pH 3, pH 4, and pH 5), actate buffer (pH 3.5, pH 4.5, and pH 5.5), phosphate buffer (pH 6.0, pH 6.5, and pH 7.0), Tris-HCl buffer (pH 7.0, pH 8.0, pH 8.5, and pH 9.0), glycine-sodium hydroxide (pH 8.5, pH 9.0, pH 9.5, pH 10, and pH 10.5), sodium borate-sodium hydroxide buffer (pH 9.5, pH 10.5, and pH 10.8), and phosphate sodium hydroxide buffer (pH 11 and pH 12). Xylanase activity of the N546b xylanase was determined with an enzyme solution (0.1 mL) having xylan-hydrolyzing activity of 0.1 units/mL, whereas CP degrading activity of the N546b xylanase was determined with an enzyme solution (0.1 mL) having a CP degrading activity of 0.25 units/mL. All activities are expressed as a percentage relative to the maximum activity (100%). When xylan was used as the substrate, the optimum reaction pH was approximately in the vicinity of pH 8.5. Between pH 8.0 and 9.0, not less than 90% of the maximum activity was exhibited, and between pH 7 and 10, not less than 60% of the maximum activity was exhibited. When CP was used as the substrate, the optimum reaction pH was approximately pH 5.5. Between pH 4.5 and 9.0, not less than 90% of the maximum activity was exhibited, and between pH 4 and 9.5, not less than 60% of the maximum activity was exhibited. Thus, the enzyme of the present invention was found to exhibit a very unique trait; i.e., different optimum pHs for different substrates (see Fig. 1).

### (3) pH Stability

The N546b xylanase was treated at 5°C for 20 hours in various buffers (0.1 M each) which were glycine-HCl buffer (pH 2.5, pH 3, and pH 3.5), citric acid - sodium citrate buffer (pH 3, pH 4, and pH 5), acetate buffer (pH 3.5, pH 4.5, and pH 5.5), phosphate buffer (pH 6.0, pH 6.5, and pH 7.0), Tris-HCl buffer (pH 7.0, pH 8.0, pH 8.5, and pH 9.0), glycine-sodium hydroxide (pH 8.5, pH 9.0, pH 9.5, pH 10, and pH 10.5), sodium borate - sodium hydroxide buffer (pH 9.5, pH 10.5, and pH 10.8), and phosphate sodium hydroxide buffer (pH 11 and pH 12). Subsequently, in each case, activity of the N546b xylanase was determined through a standard method for determining xylanase activity and a standard method for determining CPase activity described hereinlater in the Referential Examples. All activities are expressed as a residual activity (%) relative to the activity of the pH-buffer-untreated enzyme (100%). In this experiment, an enzyme solution of 0.1 U/mL (in terms of xylanase) and an enzyme solution of 0.25 U/mL (in terms of CP) were employed. The experiment results reveal that residual xylanase activity and residual CP degrading activity are both not less than 80% within a pH range of 4 to 10.5, thus proving that the enzyme is stable within a wide pH range (Fig. 2).

### (4) Optimum reaction temperature

In accordance with the standard method for determining xylanase activity and the standard method for determining CPase activity described hereinbelow under the heading Reference Examples, reaction was carried out at different reaction temperatures of 20°C, 30°C, 40°C, 50°C, 55°C, 60°C, 70°C, or 80°C. i.e., 20°C, 30°C, 40°C, 50°C, 55°C, 60°C, 70°C, and 80°C, 20°C. All activities are expressed as a relative activity (%) to the value (100%) at a temperature at which a maximum activity is attained. In the measurement of xylanase activity, an enzyme solution (0.1 mL) of 0.05 U/mL in terms of xylan degrading activity was employed, whereas in the measurement of CPase activity, an enzyme solution (0.1 mL) of 0.125 U/mL in terms of CPase activity was employed. Thus, optimum reaction temperatures of the present enzyme for reacting with xylan or CP were determined. The optimum reaction temperature that provides maximum xylanase activity and maximum CP degrading activity were found to be approximately 50°C and approximately 55°C, respectively, both centering the vicinity of 50°C (Fig. 3).

### (5) Temperature stability

An enzyme solution of 0.1 U/mL (xylanase) and an enzyme solution of 0.25 U/mL (CPase) was treated at several temperatures (30°C, 40°C, 50°C, 60°C, 70°C 80°C, 90°C) for 20 minutes in a 0.1M phosphate buffer (pH 7.0). Thereafter, residual activities were determined in accordance with the standard method for determining xylanase activity and the standard method for determining CPase activity, both described hereinlater under the heading "Referential Examples." Residual activities are expressed as a percentage relative to the activity as measured without heat treatment (100%). The measurement was performed in parallel in two systems; i.e., a system where 2mM calcium chloride was added and a system where 2mM calcium chloride was not added. Residual activities in terms of xylanase activity and CPase activity were found to remain at both 90% or more after heat treatment at 50°C for 20 minutes, proving stability of the enzyme up to 50°C. In addition, 2mM calcium chloride slightly improved stability of the enzyme against heat (see Fig. 4).

### (6) SDS-polyacrylamidegel electrophoresis

SDS-polyacrylamidegel electrophoresis was performed in accordance with the method of Laemmli et al. SDS-PAGE standard Low (Bio-Rad), which contained, as standard proteins, phosphorylase b (molecular weight: 97,400), serum albumin (molecular weight: 66,200), ovalbumin (molecular weight: 45,000), carbonic anhydrase (molecular weight: 31,000), trypsin inhibitor (molecular weight: 21,500), and lysozyme (molecular weight: 14,400), was employed as a molecular weight marker. When SDS-polyacrylamidegel electrophoresis was performed at 30 mA/gel for about 40 minutes by use of a gel having a gel concentration of 12.5%, the molecular weight of the enzyme was found to be about 40k.

### Referential Examples Methods for determining enzyme activity (1) Method for determining CMCase activity [3.5-dinitrosalicylic acid (DNS) method]

N546b xylanase was diluted so as to have an appropriate concentration, and the enzyme solution (0.1 mL) was added to a substrate solution (0.9 mL) containing 1% (w/v) carboxymethyl cellulose (CMC, product of Nippon Paper Industries Co., Ltd.) in 0.1M glycine-sodium hydroxide (pH 9.0). The mixture was allowed to react at 40°C for 20 minutes. Subsequently, a DNS solution (1 mL) was added thereto, followed by heating in a boiling water bath for 5 minutes. The mixture was cooled with ice water, and deionized water (4 mL) was added thereto. After stirring, absorbance of the mixture was measured at 535 nm by means of U2000 Spectrophotometer (Hitachi Ltd.). The amount of enzyme which allows glucose to be released at 1 µmol/min was regarded as "1 unit of enzyme."

### (2) Method for determining CPase activity (crystalline cellulose-hydrolyzing activity)

N546b xylanase was diluted so as to have an appropriate concentration, and the enzyme solution (0.1 mL) was added to a substrate solution (0.9 mL) containing 1% (w/v) cellulose powder (CP, SIGMACELL, type 101, product of Sigma) in 0.1M glycine-sodium hydroxide (pH 9.0). The mixture was allowed to react at 40°C for 1 to 5 hours. Subsequently, a DNS solution (1 mL) was added thereto, followed by centrifugation (3000 rpm, 25°C, 15 minutes). An aliquot (1 mL) of the mixture and deionized water (2 mL) were mixed under stirring. The mixture was heated in a boiling water bath for 5 minutes, followed by cooling with ice water. Subsequently, absorbance of the mixture was measured at 535 nm by means of U2000 Spectrophotometer (Hitachi Ltd.). The amount of enzyme which allows glucose to be released at 1 µmol/min was regarded as "1 unit of enzyme." The above method was employed as a standard method for determining CPase activity (crystalline-cellulose-hydrolyzing activity).

### (3) Method for determining xylanase activity

N546b xylanase was diluted so as to have an appropriate concentration, and the enzyme solution (0.1 mL) was added to a substrate solution (0.9 mL) containing 1% (w/v) xylan (Oat-spelt, product of Fluka) in 0.1M glycine-sodium hydroxide (pH 9.0). The mixture was allowed to react at 40°C for 20 minutes. Subsequently, a DNS solution (1 mL) was added thereto, followed by heating in a boiling water bath for 5 minutes. The mixture was cooled with ice water, followed by centrifugation (3000 rpm, 25°C, 15 minutes). An aliquot (1 mL) of the mixture and deionized water (4 mL) were mixed under stirring. Subsequently, absorbance of the mixture was measured at 535 nm by means of U2000 Spectrophotometer (Hitachi Ltd.). The amount of enzyme which allows xylose to be released at 1 µmol/min was regarded as "1 unit of enzyme." The above method was employed as a standard method for determining xylanase activity.

### (4) Method for determining avicelase activity

N546b xylanase was diluted so as to have an appropriate concentration, and the enzyme solution (0.1 mL) was added to a substrate solution (0.9 mL) containing 1% (w/v) AZCL-avicel (product of Megazyme) in 0.1M glycine-sodium hydroxide (pH 9.0). The mixture was allowed to react at 40°C for 4 hours. Subsequently, ethanol (3 mL) was added thereto under stirring, followed by centrifugation (3000 rpm, 25°C, 15 minutes). Absorbance of the supernatant was measured at 650 nm by means of U2000 Spectrophotometer (Hitachi Ltd.).

### (5) Method for determining chitinase activity

A substrate solution was prepared from 1% (w/v) colloidal chitin and 0.2M glycine-sodium hydroxide buffer (pH 9) such that substrate concentration and buffer concentration were adjusted to 0.4% (w/v) and 0.1 M, respectively. N546b xylanase solution (0.1 mL) was added to the thus-prepared substrate solution (0.9 mL). The mixture was allowed to react at 40°C for 2 hours. Subsequently, a DNS solution (1 mL) was added to the reaction mixture so as to terminate reaction. The mixture was boiled in a boiling water bath for 5 minutes, cooled with ice water, and centrifugated (3000 rpm, 15 minutes, 25°C). Absorbance of the supernatant was measured at 535 nm by means of a micro plate reader (VERSAmax, product of Molecular Devices). Separately, the DNS solution (1 mL) was added to the same substrate solution (0.9 mL), followed by adding crude enzyme solution (0.1 mL). The mixture was further treated in the same manner as mentioned above, whereby a blank sample was obtained. The amount of enzyme which allows N-acetylglucosamine to be released at 1 µmol/min was regarded as "1 unit of enzyme."

### (6) Method for determining mannanase activity

A solution (0.2 mL) of 1% (w/v) AZCL-galactomannan (product of Megazyme), 0.5M glycine-sodium hydroxide buffer (pH 10) (0.2 mL), deionized water (0.5 mL), and N546b xylanase solution (0.1 mL) were mixed, and the mixture was allowed to react at 40°C for 20 minutes. Subsequently, ethanol (3 mL) was added thereto, and the mixture was centrifugated (3000 rpm, 15 minutes, 25°C). Absorbance of the supernatant separated from the precipitate was measured at 600 nm. As used herein, mannanase unit (1 Man U) refers to the amount of enzyme which allows a sample obtained through the above reaction/treatment procedure to exhibit an absorbance at 600 nm of 0.44. Note that 1 unit of mannanase derived from *Bacillus* sp. AM001 strain provides an absorbance of 0.44 under the same conditions. Thus, the value 0.44 was employed as a standard.

### (7) Method for.determining β(1,3)-glucanase activity

A solution (0.2 mL) of 1% (w/v) AZCL-curdlan (β(1,3)-glucan, product of Megazyme), 0.5M glycine-sodium hydroxide buffer (pH 10) (0.2 mL), deionized water (0.5 mL), and N546b xylanase solution (0.1 mL) were mixed, and the mixture was allowed to react at 40°C for 20 minutes. Subsequently, ethanol (3 mL) was added thereto, and the mixture was centrifugated (3000 rpm, 15 minutes, 25°C). Absorbance of the supernatant separated from the precipitate was measured at 600 nm.

### Example 8 Effect on removal of "nappy" fibers-hereinafter may be referred to as fuzziness removal effect-from the surface of cotton fabric attained by combination with different cellulases

The N546 xylanase of the present invention was used in combination with respective cellulases given below, and the effect of removing fuzziness from the surface of a cotton fabric was investigated.

### (1) Method of fuzziness removal treatment

Procedures employed for fuzziness removal treatment were as follows. Knitted fabric (dark-blue, used one) for shirts was caused to have a fuzzy surface through repeated washing with a commercially available detergent. The resultant fabric was sewn so that fuzzy, or nappy, surfaces come outside, whereby test fabric pieces (6 cm x 6 cm each) were prepared. Three test fabric pieces were subjected to a Terg-o-tometer. Specifically, the test fabric pieces were treated with a thermostatically maintained processing solution (1 L, at 40°C) containing one of the following cellulases, in the presence or absence of the xylanase of the present invention at 120 rpm or more for 2 hours. After completion of the treatment, the test pieces were rinsed thoroughly with city water and dried in a dryer together with several towels.

### <Cellulase>

1) Renozyme (product of Novozyme)
2) Carezyme (product of Novozyme)
3) KSM-S237 Cellulase (JP-A-10-313859)
4) KSM-N252 Cellulase (JP-A-2001-043663)
5) KSM-N145 Cellulase (JP-A-2005-287441)

### (2) Measurement of fuzziness removal degree

Fuzziness removal performance was evaluated with a spectrocolorimeter (model CM-3500d, product of Minolta) on the basis of the L value (lightness) in Lab color space under an SCE (specular component excluded) setting. Reduction in L value (ΔL; i.e., lowering of lightness after undergoing treatment), calculated from the L value data of the samples before and after treatment, was employed as an indicator for fuzziness removal degree. In each test, ΔL was measured six times (N=6), and the obtained six values were averaged.

### (3) Results

When 0.02U KSM-N145 cellulase (enzymatic activity being measured in terms of CP degrading activity in 100 mM glycine - sodium hydroxide buffer (pH 9.0)) was employed, addition of 0.3U N546b xylanase (enzymatic activity being measured using the same buffer) to the reaction system exhibited higher fuzziness removal degree as compared with the case where KSM-N145 cellulase was employed solely (Fig. 5).

Separately, 0.01U Carezyme cellulase (enzymatic activity conditions being the same as above) was incorporated into the reaction system, and N546b xylanase (0.1U, 0.5U, or 1.0U, in terms of xylanase activity) was added thereto. As the amount of addition increased, higher fuzziness removal degree was obtained (Fig. 6).

### Example 9

The detergent compositions of the present invention were prepared by incorporating 0.1 part by weight of N546b into 100 parts by weight of the detergent compositions shown in Tables 4-1 and 4-2. When granular detergent compositions were formed, ingredients other than the enzyme, PC, AC-1, and AC-2 were first granulated to prepare a detergent base, and subsequently, the enzyme, PC, AC-1, and AC-2 were respectively granulated and blended with the detergent base granules. The thus-obtained detergent compositions have excellent detergency and are useful for washing clothes.

**Table 4-1**

| Ingredient (%) | Formulations | | | | |
|---|---|---|---|---|---|
| | 1 | 2 | 3 | 4 | 5 |
| LAS-2 | 20 | | 20.5 | | 12 |
| LAS-3 | | 15 | | | |
| AS-2 | | | 5 | | 10 |
| SAS | 3 | | | | |
| AOS | | 3 | | | |
| SFE | | 8 | | | |
| Fatty acid salt | 2 | 6 | 4 | 10 | 3 |
| AES-2 | | | | | |
| AE-3 | 3 | | | | |
| AE-4 | | 3 | 3 | 15 | |
| AE-5 | | | | | |
| AG | | | | | |
| Zeolite | 30 | 18 | 15 | 15 | |
| Oil-absorbing carrier | | | | 10 | |
| Crystalline silicate | | | | 20 | |
| Amorphous silicate | 12 | 1 | 8 | | 10 |
| STPP | | | | | 25.5 |
| Sodium carbonate | 10 | 27 | 25 | 10 | 10 |
| Pottasium carbonate | | 3 | | 2 | 5 |
| Sodium sulfite | 2 | 2 | | | 1 |
| Sodium sulfate | 4.5 | 1.5 | | 1 | 11 |
| Sodium citrate | | | 4 | 2 | |
| NTA | | | | | |
| Monoethanolamine | | | | | |
| PAA | | | | | 1 |
| AA-MA | | 3 | 3 | 5 | |
| CMC | 2 | | | | |
| PEG | 5 | 2 | 2 | 2 | 2 |
| PVP | | | | | |
| Fluorescent dye | 0.3 | 0.3 | 0.3 | 0.3 | 0.3 |
| Perfume | 0.2 | 0.2 | 0.2 | 0.2 | 0.2 |
| Water | 4 | 5 | 3 | 0.5 | 6 |
| Ethanol | | | | | |
| Propylene glycol | | | | | |
| Enzyme | 2 | 2 | 2 | 3 | 3 |
| PC | | | 3 | 3 | |
| AC-1 | | | 2 | | |
| AC-2 | | | | 1 | |
| Total | 100.0 | 100.0 | 100.0 | 100.0 | 100.0 |
| Shape | granular | granular | granular | granular | granular |

| | | | | | |
|---|---|---|---|---|---|
| LAS-2: A product obtained by neutralizing alkylbenzene sulfonic acid "Alkene L" (carbon number of the alkyl chain: 10-14; product of Nisseki Senzai Co.) with 48% NaOH. LAS-3: A product obtained by neutralizing alkylbenzene sulfonic acid "Alkene L" (carbon number of the alkyl chain: 10-14; product of Nisseki Senzai Co.) with 50% KOH. AS-2: A sodium salt of Dovanol 25 Sulfate (C12-C15 sulfuric acid)(product of Mitsubishi Kagaku Co.). SAS: Hostapur SAS93 (C13-C18 sodium alkane sulfonate) (product of Clariant Japan Co.). AOS: Sodium α-olefinsulfonate. SFE: Derived from palm oil. Sodium α-sulfo fatty acid methyl ester. Fatty acid salt: Sodium palmitate. AES-2: Polyoxyethylene alkyl (C12-C15) ether sodium sulfate (average mole number of E.O. added = 2). AE-3: Nonidet S-3 (a product obtained by adding to a C12 or C13 alcohol E.O. in an amount of three moles on average) (manufactured by Mitsubishi Kagaku Co.). AE-4: Nonidet R-7 (a product obtained by adding to a C12-C15 alcohol E.O. in an amount of 7.2 moles on average) (manufactured by Mitsubishi Kagaku Co.). AE-5: Softanol 70 (a product obtained by adding to a C12-C15 secondary alcohol E.O. in an amount of seven moles on average) (manufactured by Nippon Shokubai Co.). AG: Alkyl (palm oil-derived)glucoside (average polymerization degree: 1.5) Oil-absorbing carrier: Tixolex 25 (amorphous sodium aluminosilicate; manufactured by Kofran Chemical; oil absorption ability: 235 ml/100 g). Crystalline silicate: SKS-6; δ-Na₂Si₂O₅; crystalline laminated silicate; average grain size: 20 µm; manufactured by Clariant Japan Co. Amorphous silicate: JIS No.1 sodium silicate. STPP: Sodium tripolyphosphate. NTA: Sodium nitrilotriacetate. PAA: Sodium polyacrylate; average molecular weight: 12,000. AA-MA: Sokalan CP5; acrylic acid-maleic acid copolymer. CMC: Sunrose B1B; carboxymethylcellulose-Na; manufactured by Nihon Seishi Co.). PEG: Polyethylene glycol; average molecular weight 6,000. PVP: Polyvinylpyrrolidone; average molecular weight 40,000; K number: 26-35. Fluorescent dye: A 1:1 blend of Tinopal CBS (manufactured by Chiba Geigy) and Whitex SA (manufactured by Sumitomo Chemical Co.). (Note: In the case of liquid detergents, Tipanol CBS alone was incorporated.) Perfume: Formulated in accordance with the perfume composition described in the Examples section of JP-A-8-239700. Enzymes: A 2:1:1:1 blend of Savinase 12.0 TW (protease), Lipolase 100T (lipase), Termamyl 60T (amylase), Carezyme (cellulase)--all these are manufactured by Novo Nordisk A/S- and KAC 500 (cellulase, manufactured by Kao Co.). (Note: In the case of liquid detergents, Savinase 16.0L (protease; manufactured by Novo Nordisk A/S) alone was incorporated.) PC: Sodium percarbonate; average particle size 400 µm; coated with sodium metaborate. AC-1: TAED, tetraacetylethylene diamine; manufactured by Clariant Co., Ltd. AC-2: Sodium lauroyloxybenzene sulfonate. | | | | | |

## Claims

1. A protein described in any of the following (a), (b) and (c):
(a) a protein having an amino acid sequence of SEQ ID NO: 2;
(b) a protein which exhibits xylanase activity and has an amino acid sequence retaining 95% or higher identity with the amino acid sequence of SEQ ID NO: 2;
(c) a protein which exhibits xylanase activity and has an amino acid sequence retaining 90% or higher identity with the amino acid sequence of SEQ ID NO: 2
wherein the protein of (b) and (c) is an alkaline xylanase exhibiting the following enzymological properties:
(1) action:
hydrolyzes β-1,4-glycoside bonds of xylan;
(2) substrate specificity:
degrades xylan and crystalline cellulose;
(3) optimum pH for reaction:
when subjected to a reaction at 40°C using xylan as a substrate, optimum pH for the reaction is approximately 8.5;
(4) pH stability:
when subjected to a treatment for 20 hours at 5°C using xylan as a substrate, residual activity as measured at pH 4.0 to 10.5 is not less than 80% of the non-treatment activity;
(5) optimum reaction temperature:
when subjected to a reaction for 20 minutes at pH 9.0 using xylan as a substrate, optimum reaction temperature is about 50°C;
(6) thermal stability:
when subjected to a treatment for 20 minutes at pH 7.0 using xylan as a substrate, a residual activity of not less than 90% of the non-treatment activity is exhibited up to 50°C;
(7) molecular weight:
has a molecular weight of about 40 kDa as determined by SDS-polyacrylamide gel electrophoresis; and
(8) an optimum reaction pH of approximately 5.5 as determined in a reaction performed at 40°C using cellulose powder as a substrate.

2. A gene encoding the protein as recited in claim 1.

3. A gene comprising a polynucleotide described in any of the following (a) to (c):
(a) a polynucleotide having a nucleotide sequence of SEQ ID NO: 1;
(b) a polynucleotide which hybridizes to a polynucleotide having a nucleotide sequence of SEQ ID NO: 1 under stringent conditions, and which encodes a protein that exhibits xylanase activity; and
(c) a polynucleotide which has a nucleotide sequence retaining 80% or higher identity with the nucleotide sequence of SEQ ID NO: 1 and encodes a protein that exhibits xylanase activity,
wherein the protein of (b) and (c) is an alkaline xylanase exhibiting the following enzymological properties:
(1) action:
hydrolyzes β-1,4-glycoside bonds of xylan;
(2) substrate specificity:
degrades xylan and crystalline cellulose;
(3) optimum pH for reaction:
when subjected to a reaction at 40°C using xylan as a substrate, optimum pH for the reaction is approximately 8.5;
(4) pH stability:
when subjected to a treatment for 20 hours at 5°C using xylan as a substrate, residual activity as measured at pH 4.0 to 10.5 is not less than 80%of the non-treatment activity;
(5) optimum reaction temperature:
when subjected to a reaction for 20 minutes at pH 9.0 using xylan as a substrate, optimum reaction temperature is about 50°C;
(6) thermal stability:
when subjected to a treatment for 20 minutes at pH 7.0 using xylan as a substrate, a residual activity of not less than 90% of the non-treatment activity is exhibited up to 50°C;
(7) molecular weight:
has a molecular weight of about 40 kDa as determined by SDS-polyacrylamide gel electrophoresis; and
(8) an optimum reaction pH of approximately 5.5 as determined in a reaction performed at 40°C using cellulose powder as a substrate.

4. A recombinant vector containing a gene as recited in claim 2 or 3.

5. A transformant containing the recombinant vector as recited in claim 4.

6. The transformant as recited in claim 5, whose host is a microorganism.

7. A method of producing xylanase, comprising culturing the transformant as recited in claim 5 or 6, and collecting xylanase from a culture obtained from the culturing.

8. A detergent composition which contains the protein as recited in claim 1.

9. The detergent composition as recited in claim 8, which further comprises cellulase capable of degrading crystalline cellulose.

## Patentansprüche

1. Protein, das in einem der folgenden (a), (b) und (c) beschrieben ist:
(a) ein Protein, das eine Aminosäuresequenz der SEQ ID NO:2 hat;
(b) ein Protein, das Xylanase-Aktivität aufweist und eine Aminosäuresequenz hat, die eine Identität von 95% oder höher mit der Aminosäuresequenz der SEQ ID NO:2 beibehält;
(c) ein Protein, das Xylanase-Aktivität aufweist und eine Aminosäuresequenz hat, die eine Identität von 90% oder höher mit der Aminosäuresequenz der SEQ ID NO:2 beibehält,
wobei das Protein von (b) und (c) eine alkalische Xylanase ist, die die folgenden enzymatischen Eigenschaften aufweist:
(1) Aktivität:
hydrolysiert β-1,4-glycosidische Bindungen von Xylan;
(2) Substratspezifität:
baut Xylan und kristalline Cellulose ab;
(3) pH-Optimum für die Reaktion:
wenn einer Reaktion bei 40°C unter Verwendung von Xylan als Substrat ausgesetzt, ist das pH-Optimum für die Reaktion etwa 8,5;
(4) pH-Stabilität:
wenn einer Behandlung über 20 Stunden bei 5°C unter Verwendung von Xylan als Substrat ausgesetzt, ist die bei einem pH-Wert von 4,0 bis 10,5 gemessene Restaktivität nicht weniger als 80% der unbehandelten Aktivität;
(5) optimale Reaktionstemperatur:
wenn einer Reaktion für 20 Minuten bei einem pH-Wert von 9,0 unter Verwendung von Xylan als Substrat ausgesetzt, ist die optimale Reaktionstemperatur etwa 50°C;
(6) Temperaturstabilität:
wenn einer Behandlung für 20 Minuten bei einem pH-Wert von 7,0 unter Verwendung von Xylan als Substrat ausgesetzt, so liegt bis zu 50°C eine Restaktivität von nicht weniger als 90% der unbehandelten Aktivität vor;
(7) Molekulargewicht:
hat ein Molekulargewicht von etwa 40 kDa, wie durch SDS-Polyacrylamid-Gelelektrophorese bestimmt; und
(8) ein optimaler Reaktions-pH-Wert von etwa 5,5, wie in einer Reaktion bestimmt, die bei 40°C unter Verwendung von Cellulosepulver als Substrat durchgeführt wird.

2. Gen, das das Protein nach Anspruch 1 codiert.

3. Gen, umfassend ein Polynucleotid, das in einem der folgenden (a) bis (c) beschrieben ist:
(a) ein Polynucleotid, das die Nucleotidsequenz der SEQ ID NO:1 hat;
(b) ein Polynucleotid, das unter stringenten Bedingungen mit einem Polynucleotid, das eine Nucleotidsequenz der SEQ ID NO:1 hat, hybridisiert, und das ein Protein codiert, das Xylanase-Aktivität aufweist; und
(c) ein Polynucleotid, das eine Nucleotidsequenz hat, die eine Identität von 80% oder höher mit der Nucleotidsequenz von SEQ ID NO:1 beibehält und ein Protein codiert, das Xylanase-Aktivität aufweist,
wobei das Protein von (b) und (c) eine alkalische Xylanase ist, die die folgenden enzymatischen Eigenschaften aufweist:
(1) Aktivität:
hydrolysiert β-1,4-glycosidische Bindungen von Xylan;
(2) Substratspezifität:
baut Xylan und kristalline Cellulose ab;
(3) pH-Optimum für die Reaktion:
wenn einer Reaktion bei 40°C unter Verwendung von Xylan als Substrat ausgesetzt, ist das pH-Optimum für die Reaktion etwa 8,5;
(4) pH-Stabilität:
wenn einer Behandlung über 20 Stunden bei 5°C unter Verwendung von Xylan als Substrat ausgesetzt, ist die bei einem pH-Wert von 4,0 bis 10,5 gemessene Restaktivität nicht weniger als 80% der unbehandelten Aktivität;
(5) optimale Reaktionstemperatur:
wenn einer Reaktion für 20 Minuten bei einem pH-Wert von 9,0 unter Verwendung von Xylan als Substrat ausgesetzt, ist die optimale Reaktionstemperatur etwa 50°C;
(6) Temperaturstabilität:
wenn einer Behandlung für 20 Minuten bei einem pH-Wert von 7,0 unter Verwendung von Xylan als Substrat ausgesetzt, so liegt bis zu 50°C eine Restaktivität von nicht weniger als 90% der unbehandelten Aktivität vor;
(7) Molekulargewicht:
hat ein Molekulargewicht von etwa 40 kDa, wie durch SDS-Polyacrylamid-Gelelektrophorese bestimmt; und
(8) ein optimaler Reaktions-pH-Wert von etwa 5,5, wie in einer Reaktion bestimmt, die bei 40°C unter Verwendung von Cellulosepulver als Substrat durchgeführt wird.

4. Rekombinanter Vektor, der ein Gen nach Anspruch 2 oder 3 enthält.

5. Transformante, die den rekombinanten Vektor nach Anspruch 4 enthält.

6. Transformante nach Anspruch 5, deren Wirt ein Mikroorganismus ist.

7. Verfahren zur Herstellung von Xylanase, umfassend das Züchten der Transformante nach Anspruch 5 oder 6, und das Sammeln der Xylanase aus einer Kultur, die durch das Züchten erhalten wurde.

8. Detergenszusammensetzung, die das Protein nach Anspruch 1 enthält.

9. Detergenszusammensetzung nach Anspruch 8, die ferner Cellulase umfasst, die in der Lage ist, kristalline Cellulose abzubauen.

## Revendications

1. Protéine décrite dans l'un quelconque des (a), (b) et (c) suivants :
(a) une protéine ayant une séquence d'acides aminés de SEQ ID NO : 2 ;
(b) une protéine qui présente une activité xylanase et qui a une séquence d'acides aminés retenant 95 % ou plus d'identité avec la séquence d'acides aminés de SEQ ID NO : 2 ;
(c) une protéine qui présente une activité xylanase et qui a une séquence d'acides aminés retenant 90 % ou plus d'identité avec la séquence d'acides aminés de SEQ ID NO : 2,
où la protéine de (b) et (c) est une xylanase alcaline présentant les propriétés enzymologiques suivantes :
(1) action :
hydrolyse les liaisons β-1,4-glycoside du xylane ;
(2) spécificité de substrat :
dégrade le xylane et la cellulose cristalline ;
(3) pH optimal pour la réaction :
lors de la soumission à une réaction à 40 °C en utilisant du xylane en tant que substrat, le pH optimal pour la réaction est approximativement de 8,5 ;
(4) stabilité au pH :
lors de la soumission à un traitement pendant 20 heures à 5 °C en utilisant du xylane en tant que substrat, l'activité résiduelle telle que mesurée de pH 4,0 à 10,5 n'est pas inférieure à 80 % de l'activité sans traitement ;
(5) température réactionnelle optimale :
lors de la soumission à une réaction pendant 20 minutes à pH 9,0 en utilisant du xylane en tant que substrat, la température réactionnelle optimale est d'environ 50 °C ;
(6) stabilité thermique :
lors de la soumission à un traitement pendant 20 minutes à pH 7,0 en utilisant du xylane en tant que substrat, une activité résiduelle qui n'est pas inférieure à 90 % de l'activité sans traitement est présente jusqu'à 50 °C ;
(7) masse moléculaire :
a une masse moléculaire d'environ 40 kDa telle que déterminée par électrophorèse sur gel de SDS-polyacrylamide ; et
(8) un pH réactionnel optimal d'approximativement 5,5 tel que déterminé dans une réaction réalisée à 40 °C en utilisant de la poudre de cellulose en tant que substrat.

2. Gène codant pour la protéine selon la revendication 1.

3. Gène comprenant un polynucléotide décrit dans l'un quelconque des (a) à (c) suivants :
(a) un polynucléotide ayant une séquence nucléotidique de SEQ ID NO : 1 ;
(b) un polynucléotide qui s'hybride à un polynucléotide ayant une séquence nucléotidique de SEQ ID NO : 1 dans des conditions stringentes, et qui code pour une protéine qui présente une activité xylanase ; et
(c) un polynucléotide qui a une séquence nucléotidique retenant 80 % ou plus d'identité avec la séquence nucléotidique de SEQ ID NO : 1 et qui code pour une protéine qui présente une activité xylanase,
où la protéine de (b) et (c) est une xylanase alcaline présentant les propriétés enzymologiques suivantes :
(1) action :
hydrolyse les liaisons β-1,4-glycoside du xylane ;
(2) spécificité de substrat :
dégrade le xylane et la cellulose cristalline ;
(3) pH optimal pour la réaction :
lors de la soumission à une réaction à 40 °C en utilisant du xylane en tant que substrat, le pH optimal pour la réaction est approximativement de 8,5 ;
(4) stabilité au pH :
lors de la soumission à un traitement pendant 20 heures à 5 °C en utilisant du xylane en tant que substrat, l'activité résiduelle telle que mesurée de pH 4,0 à 10,5 n'est pas inférieure à 80 % de l'activité sans traitement ;
(5) température réactionnelle optimale :
lors de la soumission à une réaction pendant 20 minutes à pH 9,0 en utilisant du xylane en tant que substrat, la température réactionnelle optimale est d'environ 50 °C ;
(6) stabilité thermique :
lors de la soumission à un traitement pendant 20 minutes à pH 7,0 en utilisant du xylane en tant que substrat, une activité résiduelle qui n'est pas inférieure à 90 % de l'activité sans traitement est présente jusqu'à 50 °C ;
(7) masse moléculaire :
a une masse moléculaire d'environ 40 kDa telle que déterminée par électrophorèse sur gel de SDS-polyacrylamide ; et
(8) un pH réactionnel optimal d'approximativement 5,5 tel que déterminé dans une réaction réalisée à 40 °C en utilisant de la poudre de cellulose en tant que substrat.

4. Vecteur recombinant contenant un gène selon la revendication 2 ou 3.

5. Transformant contenant le vecteur recombinant selon la revendication 4.

6. Transformant selon la revendication 5, dont l'hôte est un micro-organisme.

7. Procédé de production de xylanase, comprenant la mise en culture du transformant selon la revendication 5 ou 6, et la récupération de la xylanase à partir d'une culture obtenue à partir de la mise en culture.

8. Composition de détergent qui contient la protéine selon la revendication 1.

9. Composition de détergent selon la revendication 8, qui comprend en outre une cellulase capable de dégrader la cellulose cristalline.


### SEQUENCE LISTING

<110> KAO CORPORATION
<120> Alkali xylanase
<130> KS0887
<150> JP 2005-216369
   <151> 2005-07-26
<160> 10
<170> PatentIn version 3.1
<210> 1
   <211> 1098
   <212> DNA
   <213> Bacillus sp. KSM-N546
<220>
   <221> CDS
   <222> (1)..(1098)
   <223>
<400> 1
   atg aag acg aga aag aaa acg aga tgg tat att gcc gta ctg ctt tgt      48
   Met Lys Thr Arg Lys Lys Thr Arg Trp Tyr Ile Ala Val Leu Leu Cys
   1               5                   10                  15
   ttt gtg ctg ttg ttg ccc gca gga ggt gcg cat gcc gca acc acg atc      96
   Phe Val Leu Leu Leu Pro Ala Gly Gly Ala His Ala Ala Thr Thr Ile
               20                  25                  30
   act tct aac gag att ggg acg cat gac ggt tat gat tat gaa ttt tgg     144
   Thr Ser Asn Glu Ile Gly Thr His Asp Gly Tyr Asp Tyr Glu Phe Trp
           35                  40                  45
   aag gac agc ggc ggt tcc ggc agc atg aca ctg aac agc ggc ggt acg     192
   Lys Asp Ser Gly Gly Ser Gly Ser Met Thr Leu Asn Ser Gly Gly Thr
       50                  55                  60
   ttc agc gcc cag tgg agc aac atc aac aac atc ctg ttc cgc aag ggc     240
   Phe Ser Ala Gln Trp Ser Asn Ile Asn Asn Ile Leu Phe Arg Lys Gly
   65                  70                  75                  80
   aaa aag ttc aac gag aca caa acc cat cag caa atc ggg aac atg tcg     288
   Lys Lys Phe Asn Glu Thr Gln Thr His Gln Gln Ile Gly Asn Met Ser
                   85                  90                  95
   atc acc tat ggc gcg aac ttc cag ccg aac ggt aat gcg tac ttg acc     336
   Ile Thr Tyr Gly Ala Asn Phe Gln Pro Asn Gly Asn Ala Tyr Leu Thr
               100                 105                 110
   gta tac ggc tgg acg gtg gat ccg ctc gtt gaa ttt tac att gtc gac     384
   Val Tyr Gly Trp Thr Val Asp Pro Leu Val Glu Phe Tyr Ile Val Asp
           115                 120                 125
   agc tgg gga aca tac cgt ccg aca ggc acc cat aaa gga acc att aac     432
   Ser Trp Gly Thr Tyr Arg Pro Thr Gly Thr His Lys Gly Thr Ile Asn
       130                 135                 140
   gtg gat ggc ggc acg tac gat att tat gag acg acc cgg gtg aac cag     480
   Val Asp Gly Gly Thr Tyr Asp Ile Tyr Glu Thr Thr Arg Val Asn Gln
   145                 150                 155                 160
   cca tcc att aaa gga acg gcg acg ttc aag cag tat tgg agt gtc cgg     528
   Pro Ser Ile Lys Gly Thr Ala Thr Phe Lys Gln Tyr Trp Ser Val Arg
                       165                     170                     175
   acg tcg aag cga acg agc ggc acg atc tcg gtc agc gag cat ttc aga        576
   Thr Ser Lys Arg Thr Ser Gly Thr Ile Ser Val Ser Glu His Phe Arg
               180                     185                     190
   gcc tgg gaa agc cgc ggc atg ccg atg ggg aaa atg tat gaa gtt gcc        624
   Ala Trp Glu Ser Arg Gly Met Pro Met Gly Lys Met Tyr Glu Val Ala
           195                     200                     205
   atg acg gta gag ggc tat cag agc agc gga agc gcg aat gtg tac agc        672
   Met Thr Val Glu Gly Tyr Gln Ser Ser Gly Ser Ala Asn Val Tyr Ser
       210                     215                     220
   aat aca ttg acc atc ggc ggg ggc aac ccg ggc ggt ggt aat ccg gga        720
   Asn Thr Leu Thr Ile Gly Gly Gly Asn Pro Gly Gly Gly Asn Pro Gly
   225                     230                     235                     240
   gga ggc acg aac ccg gga acg gtg acg aga gtc gaa gct gag agc atg        768
   Gly Gly Thr Asn Pro Gly Thr Val Thr Arg Val Glu Ala Glu Ser Met
                   245                     250                     255
   acc aaa agc ggg cag tac aca ggt aat atc agc tcg ccg ttc aat ggt        816
   Thr Lys Ser Gly Gln Tyr Thr Gly Asn Ile Ser Ser Pro Phe Asn Gly
               260                     265                     270
   gtg gcc ctg tac gcc aac aac gat tcg gtc aag tat acg cag tat ttt        864
   Val Ala Leu Tyr Ala Asn Asn Asp Ser Val Lys Tyr Thr Gln Tyr Phe
           275                     280                     285
   gcc aca agc acg cat agt ttc tcg ctg cga gga gcg tca aac aat gct        912
   Ala Thr Ser Thr His Ser Phe Ser Leu Arg Gly Ala Ser Asn Asn Ala
       290                     295                     300
   aac atg gcc cga gtc gat ctg aag atc ggc ggt cag acg aaa ggc acc        960
   Asn Met Ala Arg Val Asp Leu Lys Ile Gly Gly Gln Thr Lys Gly Thr
   305                     310                     315                     320
   ttc tac ttt ggc gga agt tat ccc gcg gtc tat aca ctt aac aat gtg       1008
   Phe Tyr Phe Gly Gly Ser Tyr Pro Ala Val Tyr Thr Leu Asn Asn Val
                   325                     330                     335
   agt cat ggc acc ggc aat cag gag att gag ctg att gtt acg gcg gat       1056
   Ser His Gly Thr Gly Asn Gln Glu Ile Glu Leu Ile Val Thr Ala Asp
               340                     345                     350
   gac ggg act tgg gac gct ttc att gac tat ttg gag atc cgc               1098
   Asp Gly Thr Trp Asp Ala Phe Ile Asp Tyr Leu Glu Ile Arg
           355                     360                     365
<210> 2
   <211> 366
   <212> PRT
   <213> Bacillus sp. KSM-N546
<400> 2
   Met Lys Thr Arg Lys Lys Thr Arg Trp Tyr Ile Ala Val Leu Leu Cys
   1               5                   10                  15
   Phe Val Leu Leu Leu Pro Ala Gly Gly Ala His Ala Ala Thr Thr Ile
               20                  25                  30
   Thr Ser Asn Glu Ile Gly Thr His Asp Gly Tyr Asp Tyr Glu Phe Trp
           35                  40                  45
   Lys Asp Ser Gly Gly Ser Gly Ser Met Thr Leu Asn Ser Gly Gly Thr
       50                  55                  60
   Phe Ser Ala Gln Trp Ser Asn Ile Asn Asn Ile Leu Phe Arg Lys Gly
   65                  70                  75                  80
   Lys Lys Phe Asn Glu Thr Gln Thr His Gln Gln Ile Gly Asn Met Ser
                   85                  90                  95
   Ile Thr Tyr Gly Ala Asn Phe Gln Pro Asn Gly Asn Ala Tyr Leu Thr
               100                 105                 110
   Val Tyr Gly Trp Thr Val Asp Pro Leu Val Glu Phe Tyr Ile Val Asp
           115                 120                 125
   Ser Trp Gly Thr Tyr Arg Pro Thr Gly Thr His Lys Gly Thr Ile Asn
       130                 135                 140
   Val Asp Gly Gly Thr Tyr Asp Ile Tyr Glu Thr Thr Arg Val Asn Gln
   145                 150                 155                 160
   Pro Ser Ile Lys Gly Thr Ala Thr Phe Lys Gln Tyr Trp Ser Val Arg
                   165                 170                 175
   Thr Ser Lys Arg Thr Ser Gly Thr Ile Ser Val Ser Glu His Phe Arg
               180                 185                 190
   Ala Trp Glu Ser Arg Gly Met Pro Met Gly Lys Met Tyr Glu Val Ala
           195                 200                 205
   Met Thr Val Glu Gly Tyr Gln Ser Ser Gly Ser Ala Asn Val Tyr Ser
       210                 215                 220
   Asn Thr Leu Thr Ile Gly Gly Gly Asn Pro Gly Gly Gly Asn Pro Gly
   225                 230                 235                 240
   Gly Gly Thr Asn Pro Gly Thr Val Thr Arg Val Glu Ala Glu Ser Met
                   245                 250                 255
   Thr Lys Ser Gly Gln Tyr Thr Gly Asn Ile Ser Ser Pro Phe Asn Gly
               260                 265                 270
   Val Ala Leu Tyr Ala Asn Asn Asp Ser Val Lys Tyr Thr Gln Tyr Phe
           275                 280                 285
   Ala Thr Ser Thr His Ser Phe Ser Leu Arg Gly Ala Ser Asn Asn Ala
       290                 295                 300
   Asn Met Ala Arg Val Asp Leu Lys Ile Gly Gly Gln Thr Lys Gly Thr
   305                 310                 315                 320
   Phe Tyr Phe Gly Gly Ser Tyr Pro Ala Val Tyr Thr Leu Asn Asn Val
   325 330 335
   Ser His Gly Thr Gly Asn Gln Glu Ile Glu Leu Ile Val Thr Ala Asp
   340 345 350
   Asp Gly Thr Trp Asp Ala Phe Ile Asp Tyr Leu Glu Ile Arg
   355 360 365
<210> 3
   <211> 29
   <212> DNA
   <213> Artificial sequence
<220>
   <221> misc_feature.
   <222> (3)..(15)
   <223> n stand for any base.
<220>
   <223> Oligonucleotide as mix PCR primer designed from the N terminal amino acid
   sequence of Bacillus sp. KSM-N546 alkaline xylanase.
<400> 3
   gcnacnacna thacnagyaa ygarathgg 29
<210> 4
   <211> 28
   <212> DNA
   <213> Artificial sequence
<220>
   <221> misc_feature.
   <222> (6)..(24)
   <223> n stand for any base.
<220>
   <223>Oligonucleotide as mix PCR primer designed from the N terminal amino acid
   sequence of Bacillus sp. KSM-N546 alkaline xylanase
<400> 4
   athacntcna aygarathgg nacncayg 28
<210> 5
   <211> 24
   <212> DNA
   <213>Artificial sequence
<220>
   <223>Oligonucleotide as PCR primer for sequencing designed from the nucleotide sequence of the decided partial alkaline xylanase gene of Bacillus sp. KSM-N546 alkaline xylanase.
<400> 5
   gaacttccag ccgaacggta atgc 24
<210> 6
   <211> 24
   <212> DNA
   <213> Artificial sequence
<220>
   <223>Oligonucleotide as PCR primer for sequencing designed from the nucleotide sequence of the decided partial alkaline xylanase gene of Bacillus sp. KSM-N546 alkaline xylanase.
<400> 6
   cgacatgttc ccgatttgct gatg 24
<210> 7
   <211> 25
   <212> DNA
   <213> Artificial sequence
<220>
   <223>Oligonucleotide as PCR primer for sequencing designed from the nucleotide sequence of the decided partial alkaline xylanase gene of Bacillus sp. KSM-N546 alkaline xylanase.
<400> 7
   cacgtacgat atttatgaga cgacc 25
<210> 8
   <211> 25
   <212> DNA
   <213> Artificial sequence
<220>
   <223>Oligonucleotide as PCR primer for sequencing designed from the nucleotide sequence of the decided partial alkaline xylanase gene of Bacillus sp. KSM-N546 alkaline xylanase.
<400> 8
   gtcgacaatg taaaattcaa cgagc 25
<210> 9
   <211> 35
   <212> DNA
   <213> Artificial sequence
<220>
   <223>Oligonucleotide as PCR primer for cloning of Bacillus sp. KSM-N546 alkaline
   xylanase gene.
<400> 9
   gatagaggaa atccagatct cggccagact ttctg 35
<210> 10
   <211> 37
   <212> DNA
   <213> Artificial sequence
<220>
   <223> Oligonucleotide as PCR primer for cloning of Bacillus sp. KSM-N546
   alkaline xylanase gene.
<400> 10
   cgttggcagt tcatgaaagc ttccatgctc caggttg 37